(19) **Europäisches Patentamt / European Patent Office / Office européen des brevets**

(11) **EP 3 443 900 A1**

(12) **EUROPEAN PATENT APPLICATION**
published in accordance with Art. 153(4) EPC

(43) Date of publication:
**20.02.2019 Bulletin 2019/08**

(51) Int Cl.:
***A61B 5/11*** (2006.01)

(21) Application number: **16898551.3**

(86) International application number:
**PCT/JP2016/061669**

(22) Date of filing: **11.04.2016**

(87) International publication number:
**WO 2017/179090 (19.10.2017 Gazette 2017/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Fujitsu Limited**
**Kawasaki-shi, Kanagawa 211-8588 (JP)**

(72) Inventors:
• **SASAMOTO, Yuki**
**Kawasaki-shi**
**Kanagawa 211-8588 (JP)**
• **INOMATA, Akihiro**
**Kawasaki-shi**
**Kanagawa 211-8588 (JP)**

(74) Representative: **Haseltine Lake LLP**
**Lincoln House, 5th Floor**
**300 High Holborn**
**London WC1V 7JH (GB)**

(54) **INFORMATION PROCESSING DEVICE, INFORMATION PROCESSING METHOD, AND INFORMATION PROCESSING PROGRAM**

(57) Quantification of a predetermined motion of a certain part is facilitated. As depicted at (1) of FIG. 1, an information processing apparatus (101) determines a conversion method for converting a waveform of a sensor ($102_a$) so as to maximize similarity between time-series first data that is the waveform of the sensor ($102_a$) and time-series second data that is a waveform of a sensor ($102_n$). Next, as depicted at (2) of FIG. 1, the information processing apparatus (101) converts the waveform of the sensor ($102_a$) on the basis of the determined conversion method. With the converted sensor waveform, the information processing apparatus (101) can extract features of the predetermined motion that are originally intended to be viewed.

FIG.1

EP 3 443 900 A1

**Description**

TECHNICAL FIELD

The present invention relates to an information processing apparatus, an information processing method, and an information processing program.

BACKGROUND ART

There is a conventional technique that, at a time of a predetermined motion such as walking or standing, quantifies the predetermined motion by obtaining from time-series data provided from sensors at parts, a feature amount indicating features of the motion, such as a walking speed, a stride length, a forward bending speed, or a standing time.
As a related conventional technique, there is, for example, a technique of calculating at least one standard feature amount on the basis of at least one or more reference-person feature amounts, calculating a conversion matrix between the standard feature amount and a subject feature amount, and converting an identification-target feature amount of identification-target motion data using the conversion matrix. There is also a technique of superimposing a candidate wave of a rhythmic period related to a rhythm movement obtained by performing pattern matching processing to body-motion signal information, and an auxiliary wave created by performing coarse graining of a movement track obtained by one or more integrations of the body-motion signal information with each other and selecting a period of the candidate wave having a peak in the auxiliary wave. There is another technique of creating a three-dimensional model of bones unique to a patient, aligning actual bones of the patient with the bone model unique to the patient, and tracking movements of the bones of the patient performing movements in a predetermined range to be compared with a database having bone movement data indicating movements of bones of patients for diagnosing damage.
There is also a technique of evaluating correlation between information obtained from an input signal and reference signal information previously stored, and switching the reference signal information to be referred to according to an input result to a positional-information input unit. There is another technique of obtaining a phase change amount for a given time with respect to each of a plurality of signals based on a repeated rhythmic movement associated with voluntary movements of a living body and calculating the ratio of the phase change amount with respect to each of the signals. There is also a technique of calculating gait information on the basis of acceleration of a body motion of a user during walking, the acceleration being transmitted from a display terminal, and the technique further including calculating a changing manner of the gait associated overtime according to a temporal condition, a positional condition, and a geographical condition designated by the display terminal, and outputting gain change information to the display terminal to be displayed thereon.
There is also a technique of detecting walking using a gyroscope sensor and quantifying the walking speed, the stride length, and the like. There is also a technique of detecting sitting and standing using an acceleration and gyroscope sensor.
Patent Document 1: Japanese Laid-Open Patent Publication No. 2011 - 177300
Patent Document 2: Japanese Laid-Open Patent Publication No. 2011-92696
Patent Document 3: Published Japanese-Translation of PCT Application, Publication No. 2012-516719
Patent Document 4: Japanese Laid-Open Patent Publication No. 2002-65640
Patent Document 5: Japanese Laid-Open Patent Publication No. 2012-55604
Patent Document 6: Japanese Laid-Open Patent Publication No. 2012-90651

Non-patent Literature 1: Paolo Fraccaro, three others, "Real-world Gyroscope-based Gait Event Detection and Gait Feature Extraction", eTELEMED 2014
Non-patent Literature 2: Van Lummel, six others, "Automated approach for quantifying the repeated sit-to-stand using one bodyfixed sensor in young and older adults", Gait & Posture Vol38, 2013, pp153-156

DISCLOSURE OF INVENTION

PROBLEM TO BE SOLVED BY THE INVENTION

However, according to the conventional techniques, it is difficult to quantify a predetermined motion for an affected part. Specifically, a movement of an affected part occurring with a predetermined motion is different from a movement of a normal part occurring with the predetermined motion. Furthermore, because a general method of obtaining a feature amount from data obtained by measuring a movement of a part assumes that the part moves normally, a correct feature amount cannot be obtained. Even if the movement of the affected part occurring with the predetermined motion is corrected to solve this problem, the affected part recovers over time and the movement of the affected part changes according to recovery. Therefore, contents of the correction have to be adjusted periodically with the change.

According to one aspect, one object of the present invention is to provide an information processing apparatus, an information processing method, and an information processing program that can easily quantify a predetermined motion for a certain part.

MEANS FOR SOLVING PROBLEM

According to one aspect of the present invention, an information processing apparatus, an information processing method, and an information processing program are proposed that determine a conversion method for converting time-series first data obtained from a sensor that measures a movement of a certain part occurring with a predetermined motion, the conversion method being determined so as to maximize similarity between the first data and time-series second data obtained from a sensor that measures a movement of a cooperating part occurring with the predetermined motion, the cooperating part moving in cooperation with the certain part, and convert the first data based on the determined conversion method.

EFFECT OF THE INVENTION

According to one mode of the present invention, an effect is achieved in that it is possible to easily quantify a predetermined motion for a certain part.

BRIEF DESCRIPTION OF DRAWINGS

FIG. 1 is an explanatory diagram depicting an example of an operation of an information processing apparatus 101 according to the present embodiment;
FIG. 2 is an explanatory diagram depicting a configuration example of a system 200;
FIG. 3 is an explanatory diagram depicting an example of hardware configuration of the information processing apparatus 101 and a data obtaining apparatus 201;
FIG. 4 is an explanatory diagram depicting a function configuration example of the system 200;
FIG. 5 is an explanatory diagram depicting an example in which a sensor waveform is converted using a phase difference or a difference value of the sensor waveform;
FIG. 6 is an explanatory diagram depicting respective processes of conversion processes;
FIG. 7 is an explanatory diagram depicting an example of a method of determining a motion expressing sensor;
FIG. 8 is an explanatory diagram depicting an example in which motion insufficiency is calculated from a motion conversion amount;
FIG. 9 is an explanatory diagram depicting another example in which the motion insufficiency is calculated from the motion conversion amount;
FIG. 10 is an explanatory diagram depicting an example in which a sensor waveform for an affected part is converted based on the motion insufficiency;
FIG. 11 is an explanatory diagram depicting an example of measures taken in a case where a motion is left-right asymmetric during walking;
FIG. 12 is an explanatory diagram (part one) depicting an effect according to the present embodiment;
FIG. 13 is an explanatory diagram (part two) depicting an effect according to the present embodiment;
FIG. 14 is a flowchart depicting an example of a conversion process procedure;
FIG. 15 is an explanatory diagram depicting one example of a manner of walking of a subject in a first example;
FIG. 16 is an explanatory diagram depicting one example of sensor waveforms during walking of a user uA in the first example;
FIG. 17 is an explanatory diagram (part one) depicting an example of determination of a motion expressing sensor in the first example;
FIG. 18 is an explanatory diagram (part two) depicting an example of determination of a motion expressing sensor in the first example;
FIG. 19 is an explanatory diagram (part one) depicting an example of calculation and accumulation of the motion conversion amount in the first example;

FIG. 20 is an explanatory diagram (part two) depicting an example of calculation and accumulation of the motion conversion amount in the first example;

FIG. 21 is an explanatory diagram depicting an example of extraction of the motion conversion amount in the first example;

FIG. 22 is an explanatory diagram depicting an example of conversion of a sensor waveform in the first example;

FIG. 23 is an explanatory diagram depicting an example of detection of a predetermined motion in the first example;

FIG. 24 is an explanatory diagram depicting an example of quantification of a sensor waveform in the first example;

FIG. 25 is an explanatory diagram depicting an example of a manner of standing of a subject in a second example;

FIG. 26 is an explanatory diagram depicting an example of sensor waveforms at the time of standing of the subject in the second example;

FIG. 27 is an explanatory diagram depicting an example of determination of a motion expressing sensor in the second example;

FIG. 28 is an explanatory diagram depicting an example of conversion of a sensor waveform in the second example; and

FIG. 29 is an explanatory diagram depicting an example of quantification of a sensor waveform in the second example.

## BEST MODE(S) FOR CARRYING OUT THE INVENTION

Embodiments of an information processing apparatus, an information processing method, and an information processing program of the present disclosure will be described in detail with reference to the accompanying drawings.

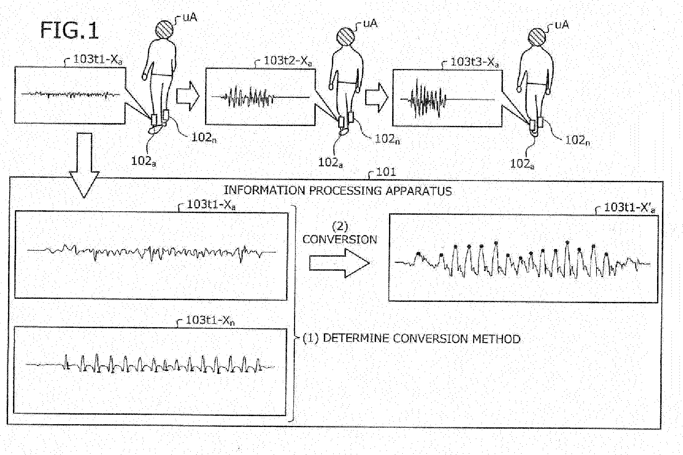

FIG. 1 is an explanatory diagram depicting an example of an operation of an information processing apparatus 101 according to the present embodiment. The information processing apparatus 101 is a computer used for quantifying a predetermined motion of a certain part, for example, an affected part. Specifically, the information processing apparatus 101 quantifies a predetermined motion of an affected part on the basis of time-series data obtained from sensors 102 attached to a patient. The predetermined motion is, for example, walking or standing.

An object to quantify a predetermined motion is, for example, to recognize the condition of a patient after discharge from a hospital or a patient being rehabilitated. Presently, with progressively increasing age of society, medical care is shifting from intensive care in a hospital to comprehensive home and community care including care at the patient's home and also care in the community overall. Associated with this, there is a growing need for a PHR (Personal Health Record) of recording various types of information, such as medical care and health information, related to individual patients in a database and managing the information in an integrated manner.

In order to seamlessly take care of a patient in a hospital and at an in-home or nursing site, it is necessary to enable recognition of the condition of the patient and transition of the condition not only in the hospital but also at home or in a nursing home. Installing a large-scale patient monitoring device to be used in a hospital at home or in a nursing home is not practical for reasons such as cost, scale, and requirements of an expert. Therefore, it is desirable that the condition of a patient can be recognized with a device relatively less expensive and easy to use.

For in this regard, a condition detecting/quantifying technique using a wearable motion sensor is considered. A motion sensor is a sensor that measures a movement of a person, an object, or the like. Specifically, a motion sensor is an acceleration sensor or a gyroscope sensor, or a combination of an acceleration sensor and a gyroscope sensor. A motion sensor is hereinafter referred to as "sensor".

For example, there is a technique of detecting walking using time-series data obtained from a sensor and quantifying a walking speed, a stride length, and the like. There is also a technique of detecting sitting and standing using time-series data obtained from a sensor. By use of these techniques, a predetermined motion of a patient can be detected and quantified as a waveform pattern formed on the basis of time-series data obtained from a sensor. Time-series data obtained from a sensor is hereinafter referred to as "sensor waveform".

However, it is difficult to quantify a predetermined motion for an affected part. Specifically, a movement of an affected part occurring with a predetermined motion is different from a movement of a normal part occurring with the predetermined motion. Furthermore, a correct feature amount cannot be obtained by a general method of obtaining a feature amount from data obtained by measuring a movement of a part because this method assumes that the part moves normally. Even if the movement of an affected part occurring with the predetermined motion is corrected to solve this problem, the affected part recovers over time and the movement of the affected part changes according to the recovery. Therefore, contents of the correction are to be adjusted periodically with the change.

For example, for a patient who drags one leg during walking due to sprain or a patient who stands while supporting the body with one hand and twisting the waist during standing due to back surgery, a movement may decelerate, have an insufficient bend, or rotate at the time of performing a predetermined motion as compared to a normal case. Accordingly, according to such change of the motion, a parameter for detecting or quantifying the motion is adjusted manually or the way or position of attachment of a sensor is corrected manually.

Further, the method of performing detection and quantification of a predetermined motion using a pattern of a sensor waveform described above assumes a movement of a specific part at normal times. Therefore, correction has to be performed for the movement of an affected part. For example, when a predetermined motion is walking, the method of performing detection and quantification of the predetermined motion using a pattern of a sensor waveform assumes the speed of rotation in a forward-backward direction at the time of walking, whereby the speed and/or timing of stepping forward, landing, and/or separating from the ground are extracted. When a predetermined motion is standing, the method of performing detection and quantification of the predetermined motion using a pattern of a sensor waveform assumes the speed of rotation in a forward-backward direction at the time of standing, whereby the speed, the timing, and/or a change in posture at the time of leaning forward are extracted.

When an assumed part is injured or diseased and becomes an affected part, the movement of the affected part at the time of performing a predetermined motion becomes different from that at normal times and a motion amount shortfall such as rotation, deceleration, or bend occurs in the movement, preventing features for quantification from being detected. For example, when the movement produced at a time of performing a predetermined motion changes, assumed features of the predetermined motion in an assumed part cannot be sensed. Furthermore, because the movement of the affected part occurring with a predetermined motion changes according to recovery, the degree of change in the movement is unknown. Therefore, there is a demand for the recognition of the degree of change in movement to be performed automatically, not manually.

Accordingly, in the present embodiment, attention is given to features of a predetermined motion being expressed on a waveform of a sensor on a part that moves in cooperation with an affected part during a predetermined motion, more specifically, a part that moves to perform the predetermined motion by joining forces with the affected part. For example, when a predetermined motion is walking and an affected part is the left leg, features of walking are expressed on a waveform of a sensor on the right leg moving in cooperation with the left leg. Therefore, data of the left leg is converted using a conversion method that maximizes the similarity between the sensor waveform of the left leg as the affected part and the sensor waveform of the normal right leg during walking.

In the following description, a sensor at which features of a motion are expressed is referred to as "motion expressing sensor". A part moving in cooperation with an affected part is referred to as "cooperating part".

An example of an operation of the information processing apparatus 101 is described with reference to FIG. 1. In the example illustrated in FIG. 1, a user uA is a patient having a sprained left leg. A sensor $102_a$ is attached to the left leg being an affected part of the user uA and a sensor $102_n$ is attached to the normal right leg. A graph 103t1-$X_a$ depicted in FIG. 1 shows a sensor waveform of an X-axis gyroscope value of the sensor $102_a$ for a given period t1. In the following graphs showing sensor waveforms, the horizontal axis represents the time and the vertical axis represents the magnitude of the value of the sensor waveform.

As shown in the graph 103t1-$X_a$, the patient uA dragging the leg due to a sprain is in a state where rotation or deceleration occurs in the movement of the left leg during walking. A graph 103t2-$X_a$ depicted in FIG. 1 shows a sensor waveform of the X-axis gyroscope value of the sensor $102_a$ for a period t2 after an elapse of a certain period from the period t1. A graph 103t3-$X_a$ depicted in FIG. 1 shows a sensor waveform of the X-axis gyroscope value of the sensor $102_a$ for a period t3 after an elapse of a certain period from the period t2. The graph 103t2-$X_a$ and the graph 103t3-$X_a$ indicate that the left leg of the user uA has recovered as compared to the respective previous periods and the sensor waveform changes according to the recovery.

In FIG. 1, an attempt is made to obtain from the graph 103t1-$X_a$, the feature amount of walking. As depicted at (1) of FIG. 1, the information processing apparatus 101 determines the conversion method of converting the waveform of the sensor $102_a$ so as to maximize the similarly between first time-series data being the waveform of the sensor $102_a$ and second time-series data being the waveform of the sensor $102_n$. A graph 103t1-$X_n$ depicted in FIG. 1 shows a sensor waveform of an X-axis gyroscope value of the sensor $102_n$ for the period t1. Black circles in the graph 103t1-$X_n$ represent peak values. Unless otherwise specified, black circles in graphs described below also represent peak values.

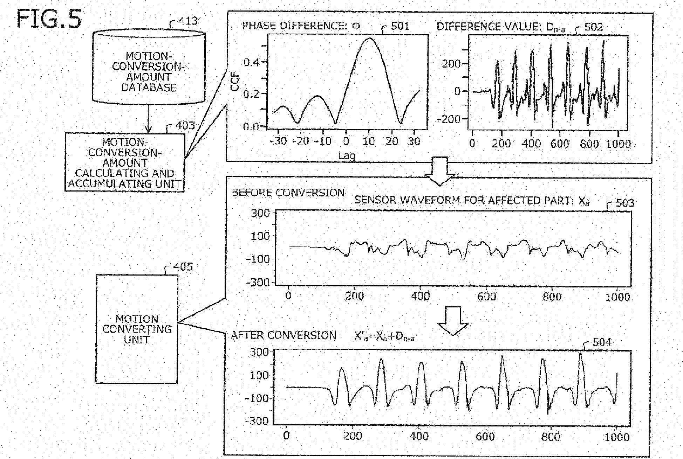

The conversion method can be, for example, a method of calculating a phase difference or a difference value in the waveforms between the sensors $102_a$ and $102_n$ and using the calculated phase difference or difference value. This method is described with reference to FIG. 5. The conversion method can alternatively be one using expression (1) as a predetermined conversion expression.

$$\hat{T} = arg \min_{T} \|U_n - TU_a\| \tag{1}$$

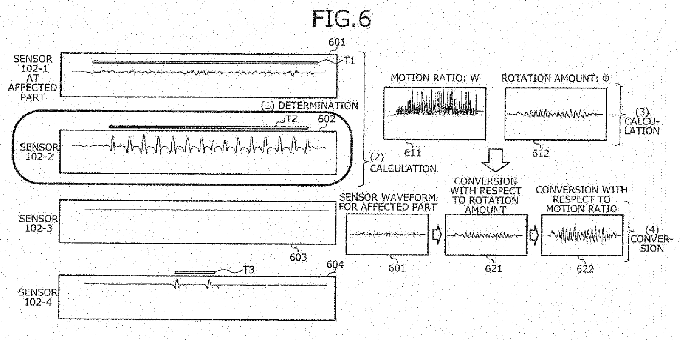

In this expression, T and T with a circumflex denote conversion matrices. Vertical double lines denote a square norm of a matrix. Furthermore, arg min f(x) denotes a function returning a value of x that minimizes f(x). $U_n$ denotes a matrix having feature amounts of a movement extracted from a waveform of a motion expressing sensor as elements. $U_a$ denotes a matrix having feature amounts of a movement extracted from a sensor waveform for an affected part as elements. Feature amounts of a movement are, for example, values themselves of a sensor waveform, peak values calculated from the sensor waveform, and a peak interval. Elements of a conversion matrix are conversion amounts of respective feature amounts and are, for example, a difference in the peak values or a rotation angle of conversion. A method using expression (1) is described with reference to FIGs. 6 to 10.

Next, the information processing apparatus 101 converts the waveform of the sensor $102_a$ on the basis of the determined conversion method as depicted at (2) of FIG. 1. A graph $103t1$-$X'_a$ depicted in FIG. 1 shows a converted sensor waveform obtained by converting the sensor waveform of the X-axis gyroscope value of the sensor $102_n$ for the given period t1 on the basis of the determined conversion method.

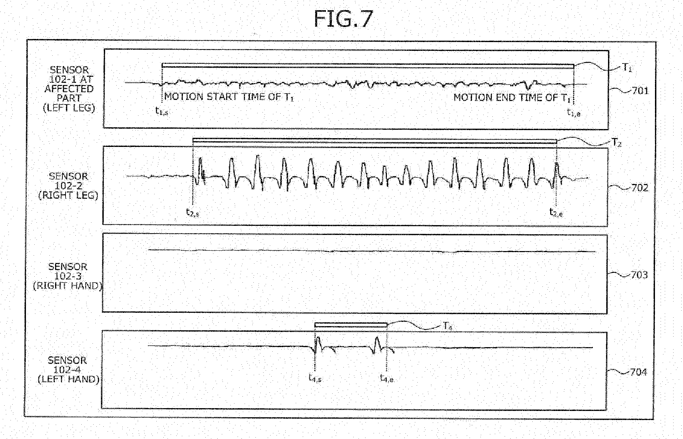

With the converted sensor waveform, the information processing apparatus 101 can extract features of a predetermined motion, which are originally intended to be viewed. If a part other than the affected part is simply randomly set as a cooperating part, erroneous conversion using a sensor waveform of a movement that is unrelated to the movement of the affected part produced with a predetermined motion may be adversely performed. An unrelated movement is a movement of the head or the upper extremities during walking. In contrast thereto, at the time of performing a predetermined motion, there is a part where a movement continues at the same time. A period in which a movement continues due to a predetermined motion is referred to as "motion duration". The information processing apparatus 101 can determine, as a cooperating part, a part having a motion duration where an overlap with the motion duration of the affected part due to a predetermined motion is long. A method of determining the cooperating part is described with reference to FIG. 7.

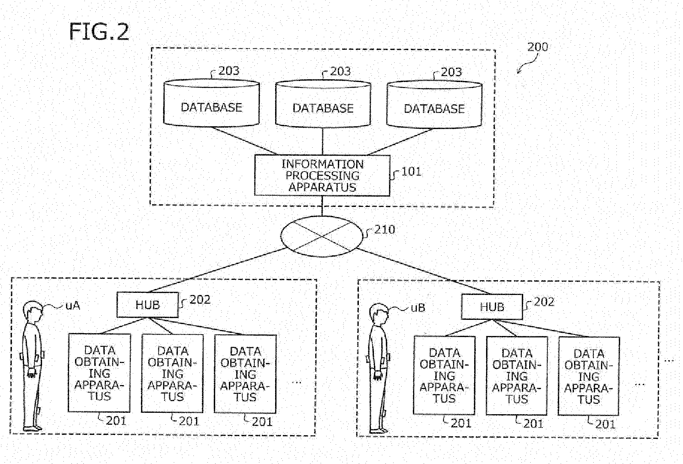

A subject to which a sensor is attached can be anything as long as it has a possibility to have an affected part. The subject is not limited to a human being but can be an animal such as a dog, a cat, or a horse. A system including the information processing apparatus 101 and the sensors 102 is described next with reference to FIG. 2.

FIG. 2 is an explanatory diagram depicting a configuration example of a system 200. The system 200 includes the information processing apparatus 101, data obtaining apparatuses 201, hubs 202, and databases 203. The information processing apparatus 101 and the data obtaining apparatuses 201 are connected via a network 210 and the hubs 202, respectively.

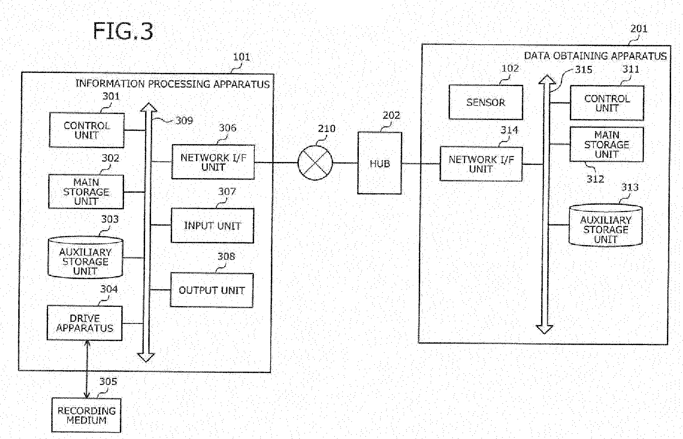

The data obtaining apparatuses 201 are devices that are attached to parts of a user and that measure movements of the respective parts. Each of the data obtaining apparatuses 201 has the sensor 102 and measures acceleration along three axes and angular speeds of the three axes of the part to which the sensor 102 is attached. In the example depicted in FIG. 3, a plurality of the data obtaining apparatuses 201 are attached to parts of the user uA and a user uB, respectively.

The databases 203 store therein data obtained from the data obtaining apparatuses 201, data obtained by converting the obtained data, and feature amounts extracted from the converted data. A hardware configuration of the information processing apparatus 101 is described next with reference to FIG. 3.

(Hardware of information processing apparatus 101 and data obtaining apparatus 201)

FIG. 3 is an explanatory diagram depicting an example of hardware configuration of the information processing apparatus 101 and the data obtaining apparatus 201. In FIG. 3, the information processing apparatus 101 includes a control unit 301, a main storage unit 302, an auxiliary storage unit 303, a drive apparatus 304, a network I/F (Interface) unit 306, an input unit 307, and an output unit 308. Further, the control unit 301 to the drive apparatus 304, and the network I/F unit 306 to the output unit 308 are respectively connected by a bus 309.

The control unit 301 is an arithmetic processing unit that governs overall control of the information processing apparatus 101. For example, the control unit 301 is a CPU (Central Processing Unit). The main storage unit 302 is a volatile memory used as a work area of the control unit 301. The volatile memory is, for example, a DRAM (Dynamic Random Access Memory), a SRAM (Static Random Access Memory), or the like.

The auxiliary storage unit 303 is a storage unit that supplements the main storage unit 302. For example, the auxiliary storage unit 303 is a HDD (Hard Disk Drive), a SSD (Solid State Drive), or the like.

The drive apparatus 304 is a control apparatus that, under the control of the control unit 301, controls the reading and writing of data with respect to a recording medium 305. For example, when the recording medium 305 is an optical disk, the drive apparatus 304 is an optical disk drive. Further, when the recording medium 305 is a magnetic disk, the drive apparatus 304 is a magnetic disk drive. The recording medium 305 is a portable recording medium. Further, the information processing apparatus 101 may have a built-in semiconductor memory formed by a semiconductor element and may read and write data from a USB (Universal Serial Bus) flash drive including a drive apparatus.

The network I/F unit 306 is connected to the network 210, which is a LAN (Local Area Network), a WAN (Wide Area Network), the Internet, etc., through a communication line and is connected to other apparatuses via the network 210. The network I/F unit 306 administers an internal interface with the network 210 and controls the input and output of data from an external apparatus. For example, a modem, a LAN adapter, etc. may be adopted as the network I/F unit 306.

The input unit 307 is an input apparatus that inputs information to the information processing apparatus 101.

For example, the input unit 307 is a mouse, a keyboard, etc. A mouse is an apparatus that moves a cursor, selects a range, or moves or changes a size of a window, etc. A keyboard has keys for inputting text, numerals, various instructions, etc., and inputs data.

The output unit 308 is an input apparatus that outputs information from the information processing apparatus 101. For example, the output unit 308 is a display, etc. A display is an apparatus that displays a cursor, an icon or a toolbox in addition to data such as documents, images, functional information, and the like. For example, a CRT (Cathode Ray Tube), a TFT (Thin Film Transistor) liquid crystal display, a plasma display, or the like can be adopted as the display.

The data obtaining apparatus 201 includes a control unit 311, a main storage unit 312, an auxiliary storage unit 313, the sensor 102, and a network I/F unit 314. The control unit 311 to the network I/F unit 314 are respectively connected via a bus 315.

The control unit 311 is an arithmetic processing unit that governs overall control of the data obtaining apparatus 201. For example, the control unit 311 is a CPU. The main storage unit 312 is a volatile memory used as a work area of the control unit 311. The volatile memory is, for example, a DRAM or an SRAM.

The auxiliary storage unit 313 is a storage unit that supplements the main storage unit 312. For example, the auxiliary storage unit 313 is an SSD.

The network I/F unit 314 is connected to the hub 202 through wireless communication or wired communication and is connected to other devices from the hub 202 via the network 210. For example, a short-range wireless communication interface can be adopted as the network I/F unit 314.

(Functional configuration example of system 200)

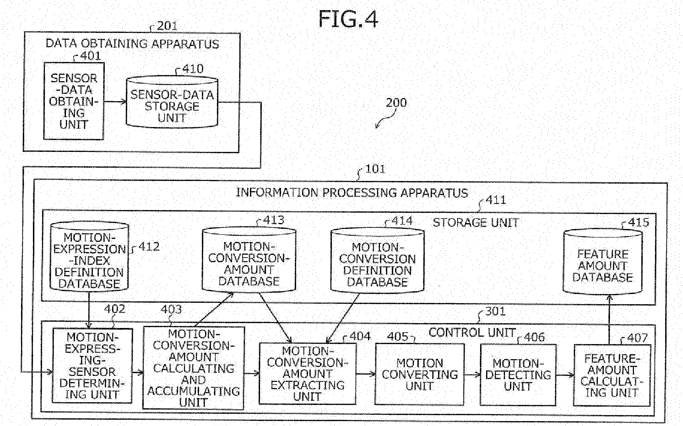

FIG. 4 is an explanatory diagram depicting a function configuration example of the system 200. The data obtaining apparatus 201 has a sensor-data obtaining unit 401. The control unit 301 includes a motion-expressing-sensor determining unit 402, a motion-conversion-amount calculating and accumulating unit 403, a motion-conversion-amount extracting unit 404, a motion converting unit 405, a motion detecting unit 406, and a feature-amount calculating unit 407. The control unit 301 executes programs stored in the main storage unit 302 and the auxiliary storage unit 303, whereby functions of the respective units are realized. Processing results of the respective units are stored to the main storage unit 302, and the like.

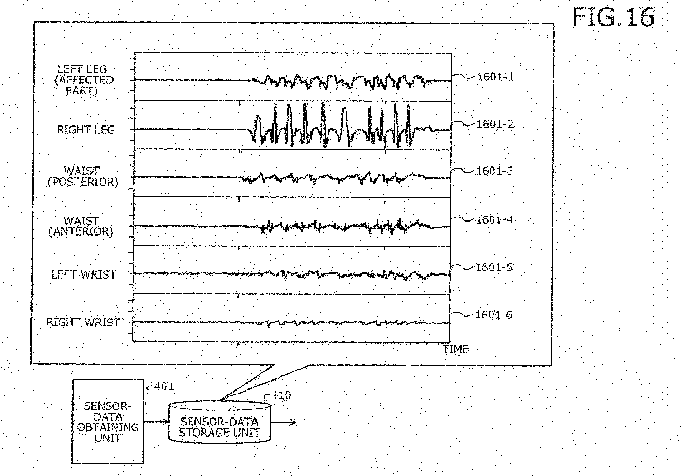

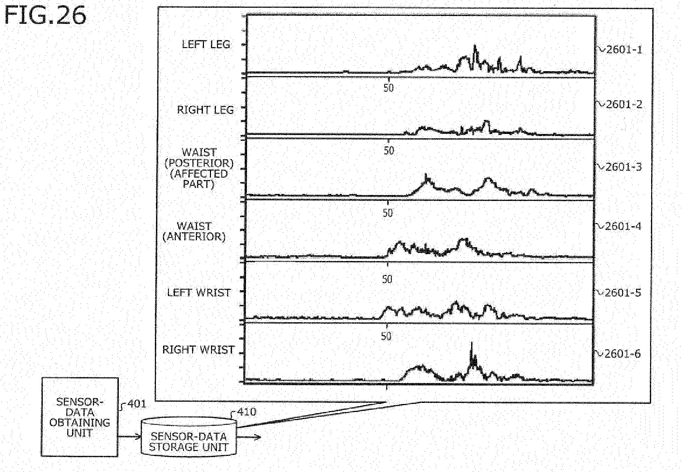

The data obtaining apparatus 201 can access a sensor-data storage unit 410. The sensor-data storage unit 410 is stored in a storage area of the main storage unit 312, the auxiliary storage unit 313, or the like. An example of storage contents of the sensor-data storage unit 410 is depicted in FIGs. 16 and 26.

The information processing apparatus 101 can access a storage unit 411 including the main storage unit 302, the auxiliary storage unit 303, and the storage medium 305. The storage unit 411 has a motion-expression-index definition database 412, a motion-conversion-amount database 413, a motion-conversion definition database 414, and a feature amount database 415.

The sensor-data obtaining unit 401 obtains sensor data measured by the sensor 102 and accumulates the obtained sensor data in the sensor-data storage unit 410.

The motion-expressing-sensor determining unit 402 determines a motion expressing sensor. As a motion expressing sensor, for example, when there are only two data obtaining apparatuses 201 attached to a user, specifically, one attached to an affected part and the other attached to a part other than the affected part, the sensor 102 of the data obtaining apparatus 201 in the part other than the affected part is determined as the motion expressing sensor.

Alternatively, it is assumed that the data obtaining apparatuses 201 are attached to a plurality of parts different from the affected part. In this case, the motion-expressing-sensor determining unit 402 can determine a cooperating part from the parts on the basis of the sensor waveform for the affected part and the sensor waveforms on the respective parts different from the affected part. At this time, the motion-expressing-sensor determining unit 402 can determine the cooperating part on the basis of a length that a period in which the affected part is moved due to a predetermined motion specified from the sensor waveform for the affected part and periods in which the respective parts are moved due to the predetermined motion specified from the sensor waveforms of the respective parts overlap with each other.

The motion-conversion-amount calculating and accumulating unit 403 determines a conversion method of converting the sensor waveform of the affected part so as to maximize the similarly between the sensor waveform of the affected part and the sensor waveform of the motion expressing sensor at the cooperating part. A specific conversion method is a method of obtaining a phase difference or a difference value in sensor data itself. This method is described with reference to FIG. 5.

The motion-conversion-amount calculating and accumulating unit 403 can alternatively determine parameter values by inputting the sensor waveform of the affected part and the sensor waveform of the motion expressing sensor to a predetermined conversion expression including parameters. The predetermined conversion expression is expression (1). The parameters are elements of a conversion matrix T. The elements are conversion amounts of respective feature amounts of a movement as described with reference to FIG. 1 and are, for example, a difference in the peak values or a rotation angle of conversion. Values of the respective elements of the conversion matrix are hereinafter referred to as "motion conversion amounts".

The motion-conversion-amount calculating and accumulating unit 403 can also calculate a deviation amount of the movement on the affected part from the predetermined motion on the basis of the accumulated motion conversion amounts. The deviation amount from the predetermined motion is referred to as "motion insufficiency".

The motion-conversion-amount calculating and accumulating unit 403 accumulates the calculated motion conversion amounts and motion insufficiencies in the motion-conversion-amount database 413.

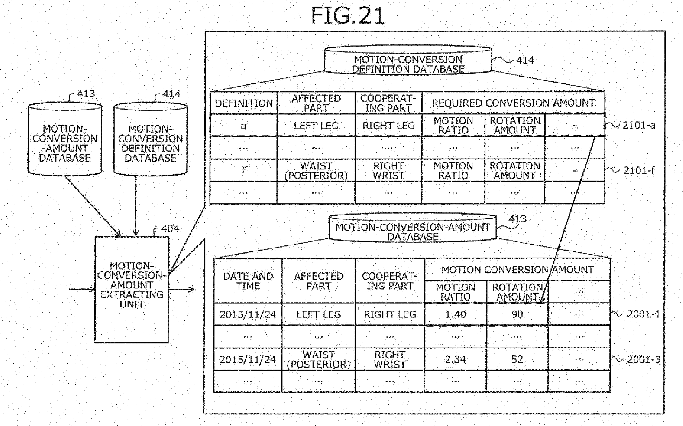

The motion-conversion-amount extracting unit 404 extracts the motion conversion amount and the motion insufficiency to be used for conversion from the motion-conversion definition database 414. A specific extraction method is described with reference to FIG. 21.

The motion converting unit 405 converts the sensor waveform for the affected part on the basis of the difference value of the sensor data itself calculated by the motion-conversion-amount calculating and accumulating unit 403. The sensor waveform for the affected part can be converted on the basis of the motion conversion amount or the motion insufficiency extracted by the motion-conversion-amount extracting unit 404.

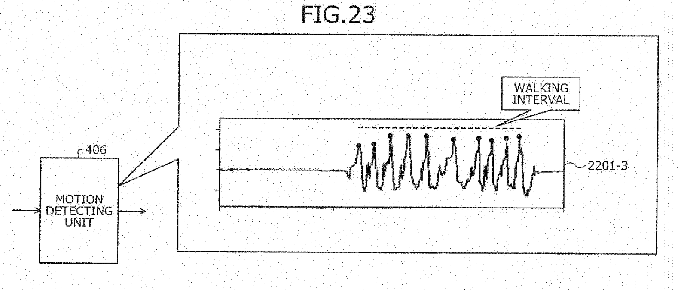

The motion detecting unit 406 detects the predetermined motion on the basis of the sensor waveform converted by the motion converting unit 405. A specific detection method is described with reference to FIG. 23.

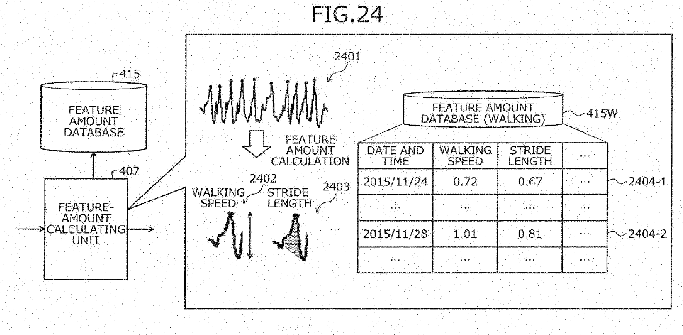

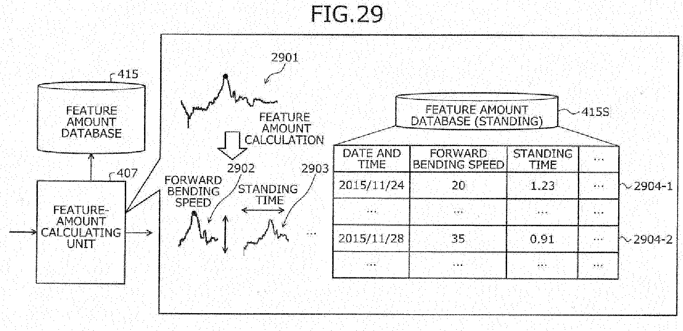

The feature-amount calculating unit 407 calculates a feature amount indicating features of the predetermined motion on the basis of the sensor waveform converted by the motion converting unit 405. A specific calculation method is described with reference to FIGs. 24 and 29.

FIG. 5 is an explanatory diagram depicting an example in which a sensor waveform is converted using a phase difference or a difference value of the sensor waveform. The motion-conversion-amount calculating and accumulating unit 403 calculates a phase difference or a difference value of the sensor data itself as the motion conversion amount. The phase difference is a correlation function value between the sensor waveform of the affected part and the sensor waveform of the cooperating part. A graph 501 depicted in FIG. 5 is a graph showing an example of a phase difference $\phi$.

The motion-conversion-amount calculating and accumulating unit 403 can calculate, for example, a time when a correlation function value as indicated by expression (2) becomes maximum.

$$\Phi = \underset{T}{arg\ max}\ abs \left( \frac{1}{N} \sum_{i=1}^{T} D_a(t) \cdot D_n(t-i) \right) \qquad (2)$$

The motion-conversion-amount calculating and accumulating unit 403 can calculate, for example, a difference value $D_{n-a}$ as a difference from a value obtained by shifting a phase-difference sensor value using expression (3). A graph 502 depicted in FIG. 5 is a graph showing an example of $D_{n-a}$.

$$D_{n-a}(t) = D_a(t) \cdot D_n(t-\Phi) \qquad (3)$$

Next, the motion converting unit 405 converts the sensor waveform of the affected part on the basis of the calculated difference value. A graph 503 depicted in FIG. 5 shows a sensor waveform $X_a$ of the affected part. The motion converting unit 405 converts the sensor waveform $X_a$ on the affected part using expression (4).

$$X'_a = X_a + D_{n-a} \qquad (4)$$

A graph 504 depicted in FIG. 5 shows a converted sensor waveform $X'_a$ obtained by converting the sensor waveform $X_a$ of the affected part.

A series of conversion processes using expression (1) among the processes described with reference to FIG. 4 is described with reference to FIG. 6.

FIG. 6 is an explanatory diagram depicting respective processes of the conversion processes. The information processing apparatus 101 performs four main procedures as the conversion processes. The information processing apparatus 101 determines the motion expressing sensor as a first procedure as depicted at (1) of FIG. 6. In the example of FIG. 6, a graph 601 showing a waveform of a sensor 102-1 at the affected part and graphs 602 to 604 respectively showing waveforms of sensors 102-2 to 102-4 at parts other than the affected part are depicted. The information processing apparatus 101 determines a cooperating part on the basis of motion durations T in which movements on the affected part or other parts continue when a certain patient performs a motion as shown by the graphs 601, 602, and 604. In the example of FIG. 6, the information processing apparatus 101 determines the sensor 102-2 as the motion expressing sensor.

Next, as a second procedure, the information processing apparatus 101 calculates a motion conversion amount for the affected part so as to maximize the similarly between the sensor waveform for the affected part and the waveform of the motion expressing sensor and accumulates the calculated motion conversion amount as depicted at (2) of FIG. 6.

As a third procedure, the information processing apparatus 101 calculates a motion insufficiency for the affected part, such as rotation, deceleration, or bend of a motion relative to the part to which the motion expressing sensor is attached, from the motion conversion amount as depicted at (3) of FIG. 6. In the example of FIG. 6, graphs 611 and 612 showing a calculated motion ratio W and a rotation amount $\phi$ as the motion insufficiency to be calculated are depicted.

Next, as a fourth procedure, the information processing apparatus 101 converts the sensor waveform for the affected part on the basis of the calculated motion insufficiency as depicted at (4) of FIG. 6. In the example of FIG. 6, a graph 621 showing a waveform obtained by performing conversion with respect to the rotation amount $\phi$ to the sensor waveform for the affected part, and a graph 622 showing a waveform obtained by performing conversion with respect to the motion ratio W to the converted sensor waveform for the affected part obtained by performing the conversion with respect to the rotation amount $\phi$ are depicted.

The procedures described with reference to FIG. 6 are described next with reference to FIGs. 7 to 10. A method of determining the motion expressing sensor is described first with reference to FIG. 7.

(Method of determining motion expressing sensor)

FIG. 7 is an explanatory diagram depicting an example of the method of determining the motion expressing sensor. The motion-expressing-sensor determining unit 402 determines the sensor 102 of a data obtaining apparatus 201 having a sensor waveform pattern, as the motion expressing sensor by a method described below.

For example, the motion-expressing-sensor determining unit 402 can define a specific pattern in advance and determine a sensor 102 having a sensor waveform most matching the defined pattern as the motion expressing sensor. The motion-expressing-sensor determining unit 402 can alternatively determine, as the motion expressing sensor, a sensor 102 having a sensor waveform where the number of times of occurrence of a movement is the same as that of a movement on the affected part due to the motion or is a multiple number thereof.

The motion-expressing-sensor determining unit 402 can determine a part where an overlapping period with the motion duration of the affected part is long, as a cooperating part and determine, as the motion expressing sensor, a sensor 102 attached to the cooperating part. The motion-expressing-sensor determining unit 402 can determine the motion duration by performing threshold processing of a composite value of the sensors 102. The motion-expressing-sensor determining unit 402 can determine, as the cooperating part, a part to which a data obtaining apparatus 201 having a sensor waveform in which the overlapping period is the longest is attached. For example, the motion-expressing-sensor determining unit 402 determines the cooperating part using expression (5).

$$S = \underset{n}{arg\ max}(Intersect(T_1, T_n)) \tag{5}$$

In this expression, S denotes the ID of the sensor 102. Further, arg max f(x) denotes a function returning a value of x that maximizes f(x). That is, expression (5) returns n that maximizes $Intersect(T_1, T_n)$. Furthermore, $Intersect(T_1, T_n)$ is defined by expression (6).

$$Intersect(T_1, T_n) = [t_s, t_e] \tag{6}$$

Where, $t_s = max(t_{a,s}, t_{b,s})$ and $t_e = max(t_{a,e}, t_{b,e})$.

FIG. 7 depicts a graph 701 showing a waveform of the sensor 102-1 of a data obtaining apparatus 201-1 attached to the affected part and graphs 702 to 704 showing waveforms of the sensors 102-2 to 102-4 of data obtaining apparatuses 201-2 to 201-4 attached to parts other than the affected part, respectively. In the example of FIG. 7, the affected part having the data obtaining apparatus 201-1 attached thereto is the left leg. The parts having the data obtaining apparatuses 201-2 to 201-4 attached thereto are the right leg, the right hand, and the left hand, respectively.

The motion-expressing-sensor determining unit 402 sets the motion duration of the affected part to $T_1$ by the threshold processing described above. In this example, the start time of $T_1$ is $t_{1,s}$ and the end time of $T_1$ is $t_{1,e}$. Similarly, the motion-expressing-sensor determining unit 402 sets the motion duration of the right leg to $T_2$ by the threshold processing. In this example, the motion start time of $T_2$ is $t_{2,s}$ and the motion end time of $T_2$ is $t_{2,e}$. The motion-expressing-sensor determining unit 402 sets the motion duration of the left hand to $T_4$ by the threshold processing. In this example, the motion start time of $T_4$ is $t_{4,s}$ and the motion end time of $T_4$ is $t_{4,e}$.

The motion-expressing-sensor determining unit 402 determines that S=2, that is, determines the right leg as the cooperating part using expression (5). An example of calculation of the motion conversion amount is described next.

(Example of calculation of motion conversion amount)

Maximizing the similarly is obtaining a conversion matrix by which features of a certain sensor waveform become most similar to features of another sensor waveform. For example, the motion-conversion-amount calculating and accumulating unit 403 calculates the motion conversion amount using expression (1) described above.

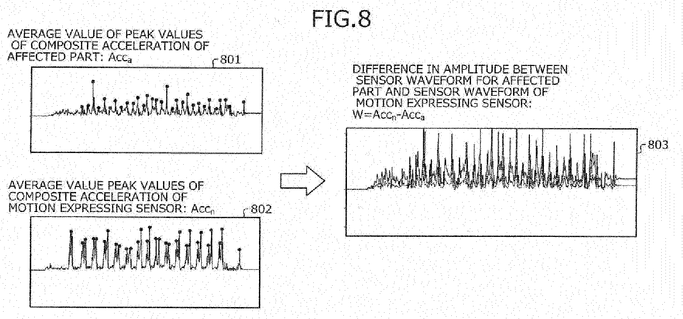

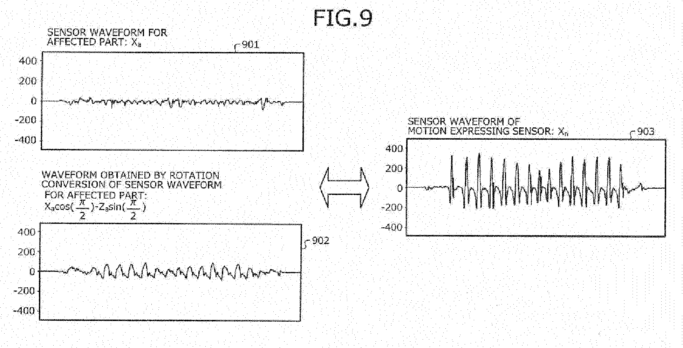

Two examples of calculation of the motion insufficiency are described next with reference to FIGs. 8 and 9.

(Examples of calculation of motion insufficiency)

FIG. 8 is an explanatory diagram depicting an example in which the motion insufficiency is calculated from the motion conversion amount. Deceleration or bend of a motion is determined, for example, from the motion ratio being one element of the motion insufficiency. Accordingly, in FIG. 8, an example in which the motion ratio is calculated is described. The motion ratio is, for example, a difference between amplitudes of the sensor waveforms. The amplitude can be, for example, a peak value of composite acceleration, an average value of the peak values, or an area.

In the example of FIG. 8, an example in which the motion ratio is calculated using peak values of the composite acceleration as the amplitude is depicted. Graphs 801 and 802 depicted in FIG. 8 are graphs related to an average value $Acc_a$ of peak values of composite acceleration of the affected part and an average value $Acc_n$ of peak values of composite acceleration of the motion expressing sensor, respectively. Points on the graphs 801 and 802 indicate peak values, respectively. For example, the motion-conversion-amount calculating and accumulating unit 403 calculates $Acc_a$ using expression (7).

$$Acc_a = \frac{1}{p_a} \sum_{i=0}^{p_a} peak_i \qquad (7)$$

In this expression, $peak_i$ denotes an i-th peak value and $p_a$ denotes the number of peaks in the sensor waveform for the affected part. Further, $peak_i$ is the motion conversion amount being one element of the conversion matrix T. Similarly, the motion-conversion-amount calculating and accumulating unit 403 calculates the average value $Acc_n$ using expression (8).

$$Acc_n = \frac{1}{p_n} \sum_{i=0}^{p_n} peak_i \qquad (8)$$

In this expression, $p_n$ denotes the number of peaks in the waveform of the motion expressing sensor. The motion-conversion-amount calculating and accumulating unit 403 calculates a difference W in the amplitude between the sensor waveform for the affected part and the waveform of the motion expressing sensor using expression (9).

$$W = Acc_n - Acc_a \qquad (9)$$

A graph 803 depicted in FIG. 8 is a graph showing the difference W. A sensor waveform shown with a broken line in the graph 803 is a sensor waveform $X_a$ before conversion. A sensor waveform shown with a solid line in the graph 803 is a sensor waveform $X_a$ after conversion using the difference W.

FIG. 9 is an explanatory diagram depicting another example in which the motion insufficiency is calculated from the motion conversion amount. Rotation of a motion is determined, for example, from the rotation amount being one element of the motion insufficiency. Accordingly, in FIG. 9, an example in which the rotation amount is calculated is described. The rotation amount is, for example, estimated through calculation of a rotation angle that minimizes a DTW (Dynamic Time Warping) distance in the sensor waveforms between the motion expressing sensor and the affected part. For example, the motion-conversion-amount calculating and accumulating unit 403 calculates the rotation angle using expression (10).

$$\hat{\Phi} = arg\ \min_{\Phi}\{DTW\{(X_a cos\Phi - Z_a sin\Phi), X_n\} + DTW\{(X_a sin\Phi - Z_a cos\Phi), Z_n\}\}$$

$$(10)$$

In this expression, $\phi$ and $\phi$ with a circumflex denote rotation angles, respectively. DTW() denotes a function for obtaining the DTW distance. Further, $X_a$ and $Z_a$ denote angular velocities in an X-axis direction and a Z-axis direction on the affected part, respectively. Furthermore, $\phi$ is the motion conversion amount being one element of the conversion matrix T. Graphs 901, 902, and 903 depicted in FIG. 9 show the sensor waveform $X_a$ for the affected part and a waveform $X_a cos(\pi/2)-Z_a sin(\pi/2)$ obtained by performing rotation conversion by $(\pi/2)$ to the sensor waveform for the affected part, respectively. The motion expressing sensor waveform $X_n$ is shown.

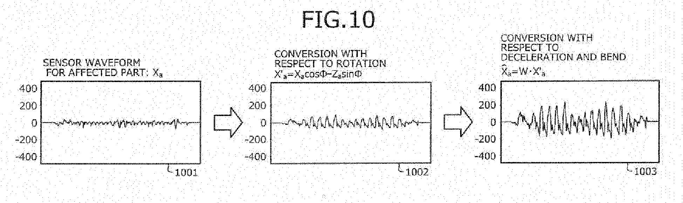

An example of conversion of the sensor waveform for the affected part is described next with reference to FIG. 10.

(Example of conversion of sensor waveform for affected part)

FIG. 10 is an explanatory diagram depicting an example in which the sensor waveform for the affected part is converted based on the motion insufficiency. The motion converting unit 405 converts the sensor waveform for the affected part on the basis of the calculated motion insufficiency such as rotation, deceleration, or bend.

A graph 1001 depicted in FIG. 10 shows the sensor waveform for the affected part. The motion converting unit 405 performs conversion with respect to rotation to the sensor waveform $X_a$ for the affected part using expression (11).

$$X'_a = X_a cos\Phi - Z_a sin\Phi \qquad (11)$$

In this expression, $X'_a$ denotes the converted sensor waveform for the affected part, obtained by the conversion with respect to rotation and $\phi$ denotes the rotation angle calculated in FIG. 9. A graph 1002 depicted in FIG. 10 shows a converted sensor waveform for the affected part, obtained by the conversion with respect to rotation. Next, using expression (12), the motion converting unit 405 performs conversion with respect to deceleration and bend to the converted sensor waveform $X'_a$ for the affected part after the conversion with respect to rotation.

$$\hat{X}_a = W \cdot X'_a \qquad (12)$$

In this expression, $X_a$ with a circumflex denotes a converted sensor waveform for the affected part, obtained by the conversion with respect to deceleration and bend. W denotes the motion ratio calculated in FIG. 8. A graph 1003 depicted in FIG. 10 shows a converted sensor waveform for the affected part, obtained by the conversion with respect to deceleration and bend.

This completes the description of the respective procedures described with reference to FIG. 6. An additional method to the respective procedures described with reference to FIG. 6 is described next. A method of restricting the cooperating part for the method of determining the cooperating part in determination of the motion expressing sensor is described first. When walking is to be detected using the data obtaining apparatuses 201 attached to both legs, the motion-expressing-sensor determining unit 402 can use either the left or right leg as the cooperating part. When standing is to be detected using the data obtaining apparatus 201 attached to the waist, a wrist or a leg can be used as the cooperating part. When there is a plurality of candidates for the cooperating part, the motion-expressing-sensor determining unit 402 can use, as the cooperating part, a part having a motion amount that is largest among the candidates for the cooperating part.

A method of restricting the conversion method is described next. When walking is to be detected by attaching the sensors 102 to the legs, the motion-conversion-amount calculating and accumulating unit 403 and the motion converting unit 405 can convert a deviation on a horizontal plane based on a reference motion on the basis that a direction in which rotation occurs due to an abnormal movement is mainly on the horizontal plane. Specifically, the motion-conversion-amount calculating and accumulating unit 403 and the motion converting unit 405 assume a rotation plane in expression (10) or (11) as the horizontal plane based on a reference motion. The part of a leg to which the sensor 102 is attached is ankle, toe, shin, or the like.

When standing is to be detected by attaching the sensor 102 to the waist, the motion-conversion-amount calculating and accumulating unit 403 and the motion converting unit 405 can perform conversion based on a motion of forward/backward rotation of the wrist or a leg on the basis that the wrist or a leg assists a forward leaning motion.

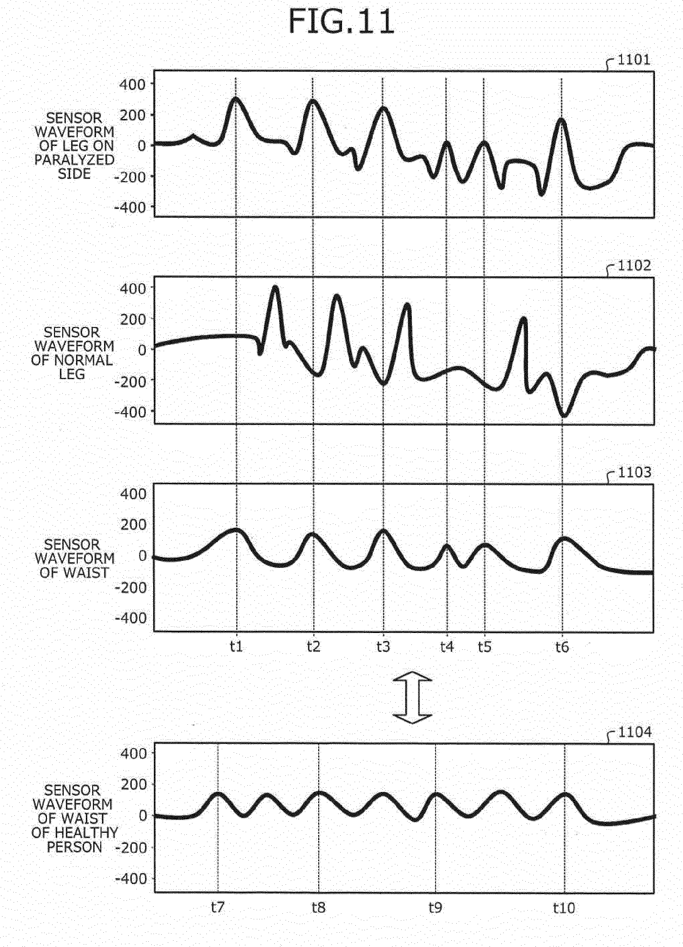

There are cases where walking is detected using the sensors 102 on the both legs for, as a target, a disease that causes semi-permanent abnormality in the motion of one leg such as hemiparesis. In this case, a correlation between the motion of one leg having a symptom as an affected part and the motion of the other normal leg being the cooperating part may be low, that is, the motion may be left-right asymmetric. Measures to be taken in this case are described with reference to FIG. 11.

FIG. 11 is an explanatory diagram depicting an example of measures taken in a case where a motion is left-right asymmetric during walking. When a motion is left-right asymmetric during walking, the motion-conversion-amount calculating and accumulating unit 403 can calculate the rotation amount as one of the motion conversion amounts from the sensor waveform for the waist. The reason why the waist is focused on is that up-and-down movements of the center of mass, that is, the waist are smaller when a leg having a symptom supports the body than when the normal leg supports the body. More specifically, it is difficult for a leg having a symptom to support the body with the knee bent. Therefore, up-and-down movements of the center of mass being generally likely to occur do not occur when a patient takes a step with a normal leg. That is, up-and-down movements of the center of mass occur when a patient takes a step with a leg having a symptom.

Graphs 1101 to 1103 in FIG. 11 show a sensor waveform of a leg on a paralyzed side of a patient, a sensor waveform of a normal leg of the patient, and a sensor waveform of the waist of the patient, respectively. A graph 1104 in FIG. 11 shows a sensor waveform of the waist of a healthy person.

At times t1 to t6 in the graphs 1101 to 1103, the patient takes steps with the leg on the paralyzed side. As shown by the graphs 1101 to 1103, the sensor waveform of the waist has peaks when the patient takes steps with the leg on the paralyzed side and the sensor waveform of the waist does not have peaks when the patient takes steps with the normal leg. As indicated at the times t4 and t5, the patient sometimes takes consecutive steps with the leg on the paralyzed side and the motion of the legs is left-right asymmetric.

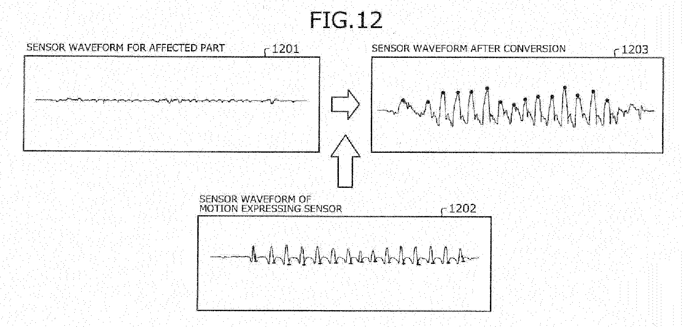

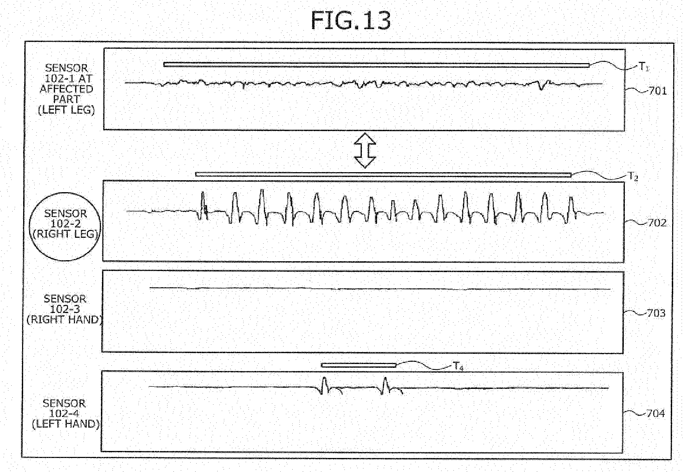

In contrast thereto, at times t7 to t10 in the graph 1104, the healthy person takes steps with one of the legs. The graph 1104 shows that the up-and-down movements occur when the person takes steps with either the left or right leg. Effects according to the present embodiment are described next with reference to FIGs. 12 and 13.

FIG. 12 is an explanatory diagram (part one) depicting an effect according to the present embodiment. The information processing apparatus 101 converts the sensor waveform for an affected part on the basis of the waveform of the motion expressing sensor even when the motion of the affected part of a patient changes according to the course of progress. This enables the information processing apparatus 101 to perform detection and quantification to extract feature points for recognizing a motion without periodically correcting the sensor waveform.

A graph 1201 depicted in FIG. 12 shows a sensor waveform for an affected part of a certain patient. Feature points of walking cannot be extracted from the graph 1201. A graph 1202 depicted in FIG. 12 shows a waveform of a motion expressing sensor on the patient. The information processing apparatus 101 converts the sensor waveform for the affected part of the patient on the basis of the waveform of the motion expressing sensor on the patient. A graph 1203 depicted in FIG. 12 shows a sensor waveform after the conversion. From the sensor waveform after the conversion, feature points of walking can be extracted.

FIG. 13 is an explanatory diagram (part two) depicting an effect according to the present embodiment. The information processing apparatus 101 automatically determines a part for a motion expressing sensor, that is, a cooperating part on the basis of an overlap between motion durations of respective parts. Graphs 701 to 704 depicted in FIG. 13 are substantially identical to those depicted in FIG. 7. In the example of FIG. 13, the information processing apparatus 101 determines the right leg having the sensor 102-2 attached thereto as the cooperating part.

Accordingly, for example, when the affected part is one leg, it is possible to find the cooperating part at which features of a motion are expressed more and that works in cooperation with the affected part, instead of previously determining a sensor attached to the other leg as the motion expressing sensor. By using the sensor waveform of the cooperating part, the information processing apparatus 101 can perform conversion with higher accuracy.

For example, if the motion expressing sensor is previously determined, a part having the previously-determined sensor attached thereto may perform a movement unrelated to a movement of the affected part occurring with a predetermined motion, and erroneous conversion may be performed in this case. At the time of performing a predetermined motion, there is a part continuing a motion at the same time. Therefore, the information processing apparatus 101 can perform conversion with higher accuracy by determining, as the cooperating part, a part having a motion duration in which an overlap with the motion duration of the affected part is long.

The information processing apparatus 101 can use the accumulated motion conversion amount as a motion quantification result. For example, a user of the information processing apparatus 101 can know the degree of recovery of a patient by utilizing the accumulated motion conversion amount as information for recognizing how the motion conversion amount changes according to the course of progress.

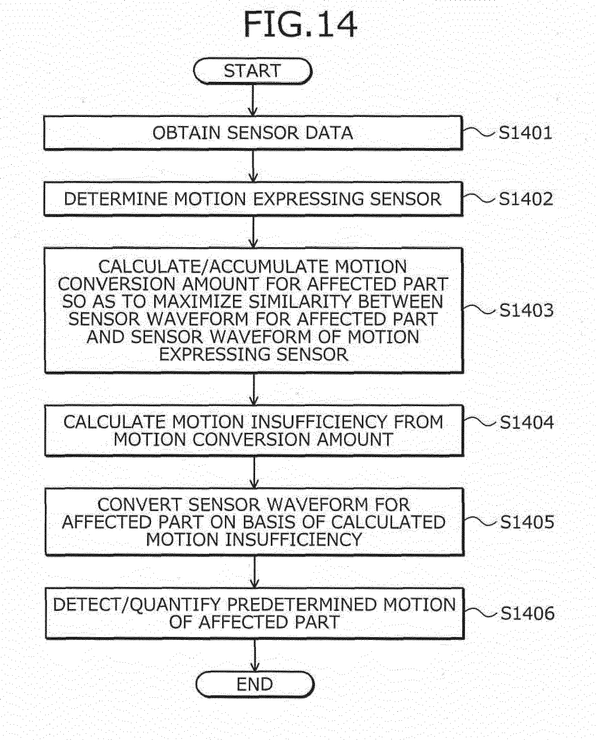

FIG. 14 is a flowchart depicting an example of a conversion process procedure. The information processing apparatus 101 obtains sensor data (step S1401). Next, the information processing apparatus 101 determines a motion expressing sensor (step S1402). Subsequently, the information processing apparatus 101 calculates the motion conversion amount for an affected part so as to maximize the similarity between a sensor waveform of the affected part and a waveform of the motion expressing sensor and accumulates the calculated motion conversion amount (step S1403).

Next, the information processing apparatus 101 calculates a motion insufficiency from the motion conversion amount (step S1404). The information processing apparatus 101 converts the sensor waveform of the affected part on the basis of the calculated motion insufficiency (step S1405). Subsequently, the information processing apparatus 101 detects and quantifies a predetermined motion of the affected part (step S1406). After the process at step S1406 ends, the information processing apparatus 101 ends the conversion processes.

An example of quantifying walking of a patient having a sprained left leg is described next as a first example of the present embodiment, and an example of quantifying standing of a patient having a paralyzed left leg is described as a second example. Therefore, a predetermined motion is walking in the first example and is standing in the second example.

(First example)

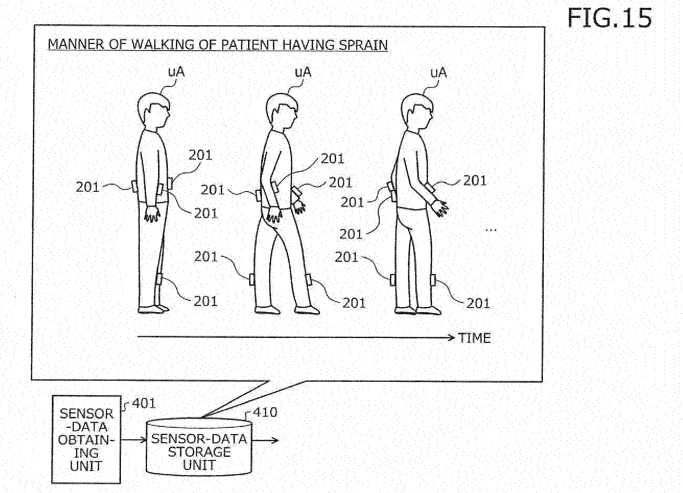

An example in which walking of a user uA having a sprained left leg is quantified is described as the first example with reference to FIGs. 15 to 24.

FIG. 15 is an explanatory diagram depicting one example of a manner of walking of a subject in the first example. In the first example, a case where the data obtaining apparatuses 201 are attached to both wrists, anteriorly and posteriorly at the waist, and the left and right legs of the user uA, respectively, is assumed. A case in which an affected part during walking of the user uA having the left leg sprained is quantified is cited as an example. FIG. 15 depicts a manner of walking of the user uA as a patient having a sprain. The sensor-data obtaining units 401 of the respective data obtaining apparatuses 201 attached to the parts of the user uA measure movements of the corresponding parts and store measured sensor waveforms to the sensor-data storage unit 410.

FIG. 16 is an explanatory diagram depicting one example of sensor waveforms during walking of the user uA in the first example. In FIG. 16, one gyroscope value of each part is depicted. Specifically, graphs 1601-1 to 1601-6 show sensor data with respect to time on the left leg, the right leg, the waist (posterior), the waist (anterior), the left wrist, and the right wrist, respectively. The sensor 102 of each of the data obtaining apparatuses 201 measures the accelerations and the gyroscope values in the three-axis directions. However, to simplify depiction, only the X-axis gyroscope value is depicted in FIG. 16. The information processing apparatus 101 obtains sensor data of each part from the corresponding sensor-data storage unit 410. The information processing apparatus 101 also sets the left leg as an affected part through an operation of a user of the information processing apparatus 101, specifically, a person in charge of treatment of the user uA.

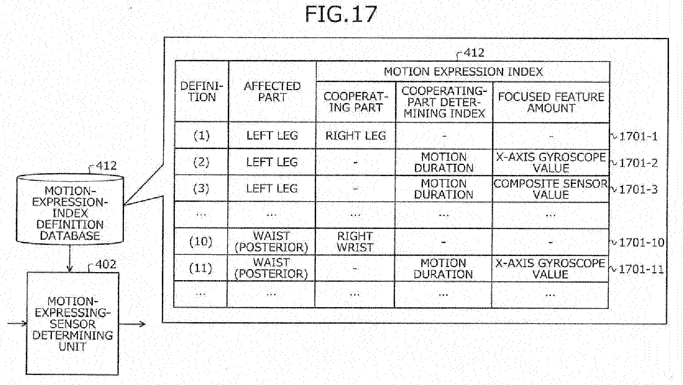

FIG. 17 is an explanatory diagram (part one) depicting an example of determination of a motion expressing sensor in the first example. The motion-expressing-sensor determining unit 402 refers to the motion-expression-index definition database 412 to determine a motion expressing sensor. The motion-expression-index definition database 412 depicted in FIG. 17 has records 1701-1 to 1701-3, 1701-10, and 1701-11.

The motion-expression-index definition database 412 includes fields for the definition, the affected part, and the motion expression index, respectively. Numbers that identify motion expression indexes are stored in the definition field. Character strings indicating affected parts are stored in the affected-part field. Information indicating a motion expression index according to an affected part is stored in the motion-expression-index field. The motion-expression-index field has subfields for the cooperating part, the cooperating-part determining index, and the focused feature amount. Names of parts having a motion expressing sensor attached thereto in a case where the parts are previously determined as the cooperating part are stored in the cooperating-part field. Character strings indicating an index for determining the cooperating part are stored as the cooperating-part determining index. Character strings indicating a feature amount to be used to determine the cooperating part are stored as the focused feature amount.

For example, the record 1701-1 shows a definition to set the right leg as the cooperating part when the affected part is the left leg. Similarly, the record 1701-2 shows a definition to specify the motion duration using X-axis sensor data of other parts and set a part having a long overlap as the cooperating part when the affected part is the left leg. The record 1701-3 shows a definition to specify the motion duration using a composite value of sensor data of other parts and to set a part having a long overlap as the cooperating part when the affected part is the left leg.

The motion-expressing-sensor determining unit 402 can use the definition of any of the records 1701-1 to 1701-3 when the affected part is the left leg. The definition of one of the records 1701-1 to 1701-3 to be used can be specified by the user of the information processing apparatus 101. In the example of FIG. 17, it is assumed that the definition of the record 1701-2 is used.

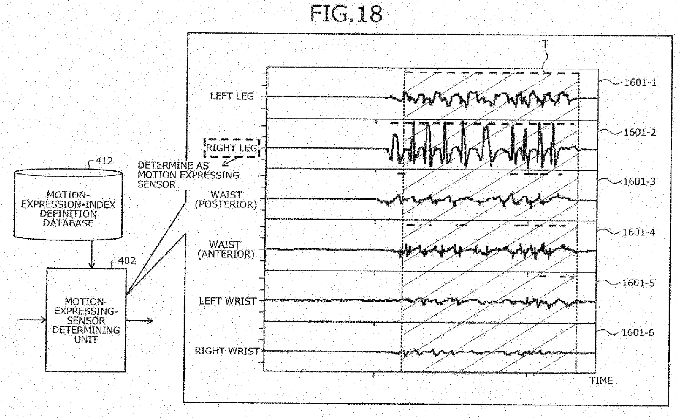

FIG. 18 is an explanatory diagram (part two) depicting an example of determination of a motion expressing sensor in the first example. The motion-expressing-sensor determining unit 402 determines a motion expressing sensor with reference to the definition of the record 1701-2. In graphs 1601-1 to 1601-6 depicted in FIG. 18, respective motion durations are indicated by broken lines. Motion durations of the left leg as the affected part are indicated by hatching. In the example of FIG. 18, the motion-expressing-sensor determining unit 402 determines that an interval $\Delta t$ is a motion duration when variance of the X-axis gyroscope value in the interval $\Delta t$ becomes equal to or smaller than a threshold.

In the example of FIG. 18, a broken line most overlapping with the hatched part is a broken line of the right leg. Therefore, the motion-expressing-sensor determining unit 402 determines the right leg as the cooperating part and sets the sensor 102 on the right leg as the motion expressing sensor.

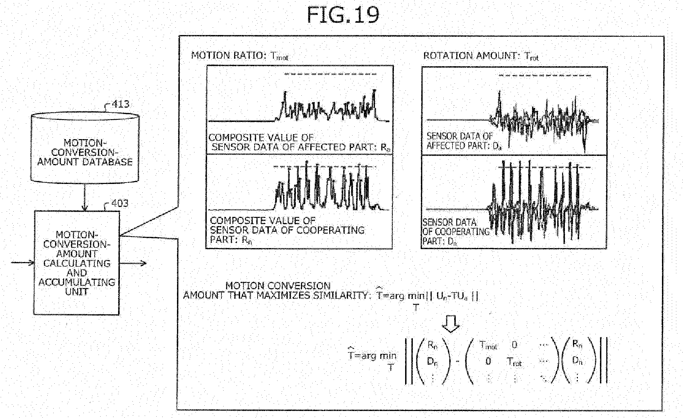

FIG. 19 is an explanatory diagram (part one) depicting an example of calculation and accumulation of the motion conversion amount in the first example. The motion-conversion-amount calculating and accumulating unit 403 calculates the motion conversion amount so as to maximize the similarity between the sensor waveform of the cooperating part and the sensor waveform of the affected part.

In the example of FIG. 19, a motion ratio $T_{mot}$ and a rotation amount $T_{rot}$ are used as elements of the conversion matrix T in expression (1). The motion ratio $T_{mot}$ is the peak ratio of the composite value of sensor data. The rotation amount $T_{rot}$ is the rotation angle. The composite value of sensor data of the affected part is denoted by $R_a$. The composite value of sensor data of the cooperating part is denoted by $R_n$. Sensor data of the affected part is denoted by $D_a$. Sensor data of the cooperating part is denoted by $D_n$.

The motion conversion amount that maximizes the similarity can be obtained from expression (1). Expression (1) can be transformed into expression (13) in the case of FIG. 19.

$$\hat{T} = \underset{T}{arg\ min}\left\|\begin{pmatrix} R_n \\ D_n \\ \vdots \end{pmatrix} - \begin{pmatrix} T_{mot} & 0 & \cdots \\ 0 & T_{rot} & \cdots \\ \vdots & \vdots & \ddots \end{pmatrix}\begin{pmatrix} R_a \\ D_a \\ \vdots \end{pmatrix}\right\| \qquad (13)$$

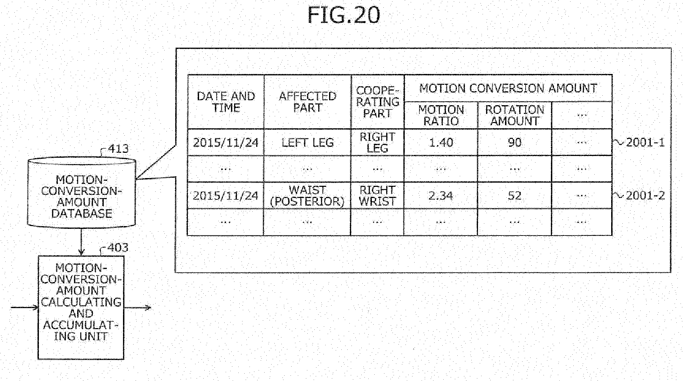

FIG. 20 is an explanatory diagram (part two) depicting an example of calculation and accumulation of the motion conversion amount in the first example. The motion-conversion-amount calculating and accumulating unit 403 accumulates the calculated motion conversion amount in the motion-conversion-amount database 413. The motion-conversion-amount database 413 depicted in FIG. 20 has records 2001-1 and 2001-2.

The motion-conversion-amount database 413 includes fields for the date and time, the affected part, the cooperating part, and the motion conversion amount. Values each indicating the date and time at which the sensors 102 performed measurement are stored in the date-and-time field. Character strings indicating the affected part are stored in the affected-part field. Character strings indicating the cooperating part corresponding to an affected part are stored in the cooperating-part field. The calculated motion conversion amounts are stored in the motion-conversion-amount field. Specifically, the motion-conversion-amount field has subfields for the motion ratio, the rotation amount, ⋯.

For example, the record 2001-1 shows that the motion ratio has been calculated to be 1.40 and the rotation amount has been calculated to be 90 from sensor data measured on November 24, 2015 with the left leg assumed as the affected part and the right leg assumed as the cooperating part.

FIG. 21 is an explanatory diagram depicting an example of extraction of the motion conversion amount in the first example. The motion-conversion-amount extracting unit 404 extracts a motion conversion amount to be used for conversion of the sensor waveform for the affected part, from the motion-conversion-amount database 413 with reference to the motion-conversion definition database 414. The motion-conversion definition database 414 depicted in FIG. 21 has records 2101-a and 2101-f.

For example, the motion-conversion-amount extracting unit 404 searches the motion-conversion definition database 414 for a record having a combination of the affected part and the cooperating part registered in the motion-conversion-amount database 413. In the example of FIG. 21, the motion-conversion-amount extracting unit 404 extracts the motion ratio W=1.40 and the rotation amount $\phi$=90 on the basis of a definition "a" shown by the record 2101-a retrieved by the search.

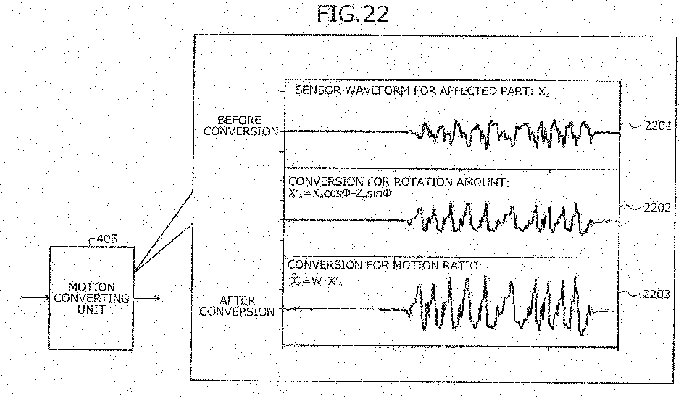

FIG. 22 is an explanatory diagram depicting an example of conversion of a sensor waveform in the first example. The motion converting unit 405 converts the sensor waveform for the affected part on the basis of the extracted motion conversion amount. A graph 2201 depicted in FIG. 22 is a graph showing a sensor waveform $X_a$ of the affected part before conversion. A graph 2202 shows a waveform $X'_a$ obtained by performing conversion for the rotation amount to the sensor waveform $X_a$ of the affected part. A graph 2203 shows a waveform $X_a$ with a circumflex, which is a waveform obtained by performing conversion for the motion ratio to the waveform $X'_a$ obtained by performing the conversion for the rotation amount. Because the conversion expression is the same as that depicted in FIG. 10, descriptions thereof are omitted.

FIG. 23 is an explanatory diagram depicting an example of detection of a predetermined motion in the first example. The motion detecting unit 406 detects walking as a predetermined motion on the basis of the converted sensor waveform. In the example of FIG. 23, a case where detection is performed by a general walking detecting algorithm is assumed. The motion detecting unit 406 detects, for example, a walking interval on the basis of peak values of the X-axis gyroscope value after conversion.

A graph 2201-3 depicted in FIG. 23 shows the waveform $X_a$ with a circumflex indicating the converted sensor waveform obtained by converting the sensor waveform for the affected part. The motion detecting unit 406 detects a walking interval on the basis of peak values of the waveform $X_a$ with a circumflex. In the example of FIG. 23, a time shown with a broken line is a walking interval.

FIG. 24 is an explanatory diagram depicting an example of quantification of a sensor waveform in the first example. The feature-amount calculating unit 407 calculates a feature amount from the sensor waveform in the detected motion interval to quantify the predetermined motion. When the predetermined motion is walking, the feature amount of walking is, for example, the walking speed or the stride length.

Reference numeral 2401 depicted in FIG. 24 denotes a sensor waveform that is in the detected motion interval and is a part of the graph 2201-3. Reference numeral 2402 denotes a part of the sensor waveform in the detected motion interval and indicates which part of the sensor waveform corresponds to the walking speed. As indicated by reference numeral 2402, the walking speed corresponds to the amplitude of the sensor waveform. Reference numeral 2403 denotes a part of the sensor waveform in the detected motion interval and indicates which part of the sensor waveform corresponds to the stride length. As indicated by reference numeral 2403, the stride length corresponds to a hatched area in the sensor waveform. The feature-amount calculating unit 407 accumulates the calculated feature amount in the feature amount database 415.

A feature amount database (walking) 415W depicted in FIG. 24 has the calculated feature amounts of walking stored therein. The feature amount database (walking) 415W depicted in FIG. 24 has records 2404-1 and 2404-2.

The feature amount database (walking) 415W includes fields for the date and time, the walking speed, the stride length, ⋯. Values indicating the date and time when the sensors 102 performed measurement are stored in the date-and-time field. Values indicating the calculated walking speed are stored in the walking-speed field. Values indicating the calculated stride length are stored in the stride-length field. The feature amount database (walking) 415W can also include the extracted motion conversion amounts such as the motion ratio and the rotation amount.

(Second example)

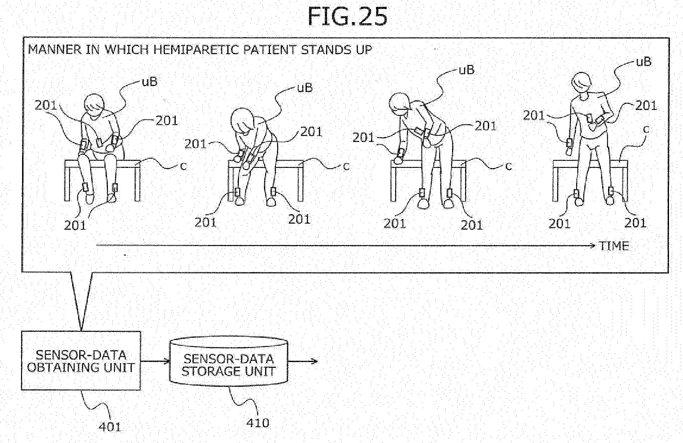

An example in which standing of a user B having a paralyzed left leg is quantified is described as a second example with reference to FIGs. 25 to 29.

FIG. 25 is an explanatory diagram depicting an example of a manner of standing of a subject in the second example. In the second example, a case where the data obtaining apparatuses 201 are attached to the both wrists, anteriorly and posteriorly at the waist, and the left and right legs of the user uB, respectively, is assumed. A case where the affected part during standing of the user uB having the paralyzed left leg is quantified is given as an example. FIG. 25 depicts the manner in which the user uB being a hemiparetic patient stands up from a state of sitting on a chair c. The sensor-data obtaining unit 401 of each of the data obtaining apparatuses 201 attached to the respective parts of the user uB measures the movement of the corresponding part and stores the measured sensor waveform in the sensor-data storage unit 410.

FIG. 26 is an explanatory diagram depicting an example of sensor waveforms at the time of standing of the subject in the second example. FIG. 26 depicts one gyroscope value for each part. Specifically, graphs 2601-1 to 2601-6 show sensor data with respect to each time at the left leg, the right leg, the waist (back), the waist (front), the left wrist, and the right wrist, respectively. The sensor of each of the data obtaining apparatuses 201 measures the accelerations or the gyroscope values in the three axis directions. However, to simplify depiction, only the X-axis gyroscope value is depicted in FIG. 26. The information processing apparatus 101 obtains sensor data of each part from the sensor-data storage unit 410. The information processing apparatus 101 sets the waist (posterior) as the affected part according to an instruction of the user of the information processing apparatus 101.

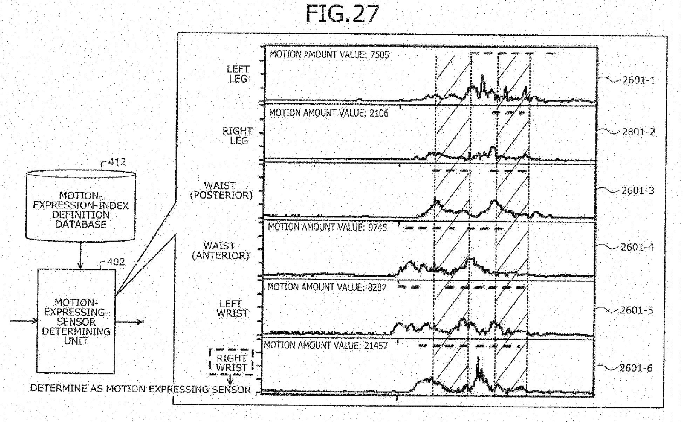

FIG. 27 is an explanatory diagram depicting an example of determination of a motion expressing sensor in the second example. The motion-expressing-sensor determining unit 402 determines a motion expressing sensor with reference to the motion-expression-index definition database 412. In graphs 2601-1 to 2601-6 depicted in FIG. 27, respective motion durations are shown with broken lines. The motion duration of the waist (posterior) as the affected part is shown as a hatched area. In the example of FIG. 27, the motion-expressing-sensor determining unit 402 determines that an interval Δt as the motion duration when variance of the X-axis gyroscope value in the interval Δt becomes equal to or smaller than a threshold.

When there are a plurality of parts having an overlap equal to or longer than a certain level in the motion duration, the motion-expressing-sensor determining unit 402 can determine a part having a largest motion amount, as the cooperating part. The motion amount is, for example, a total value of the composite accelerations in the motion duration.

In the upper left parts of the graphs 2601-1, 2601-2, and 2601-4 to 2601 - 6, one example of the motion amount value is shown. In the example of FIG. 27, the motion amount of the right wrist is the largest. Accordingly, the motion-expressing-sensor determining unit 402 determines the right wrist as the cooperating part and sets the sensor 102 on the right wrist as the motion expressing sensor. Calculation and extraction of the motion conversion amount after the motion expressing sensor is determined are performed by the same method as that in the first example. Therefore, descriptions thereof are omitted.

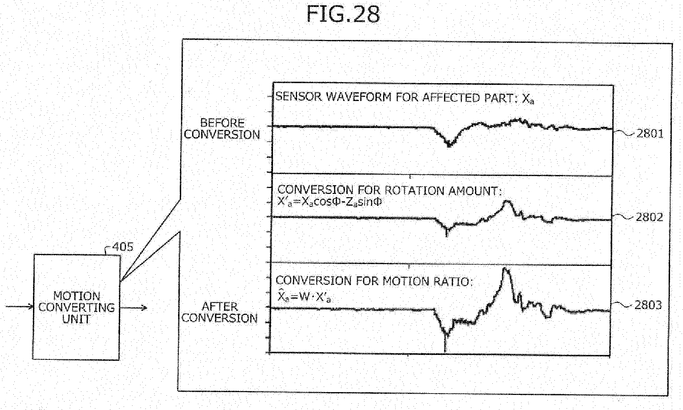

FIG. 28 is an explanatory diagram depicting an example of conversion of a sensor waveform in the second example. The motion converting unit 405 converts the sensor waveform for an affected part on the basis of the extracted motion conversion amount. A graph 2801 depicted in FIG. 28 is a graph showing a sensor waveform $X_a$ of the affected part before conversion. A graph 2802 shows a waveform $X'_a$ obtained by performing conversion for the rotation amount to the sensor waveform $X_a$ of the affected part. A graph 2803 shows a waveform $X_a$ with a circumflex, which is a waveform obtained by performing conversion for the motion ratio to the waveform $X'_a$ obtained by performing the conversion for the rotation amount. Because the conversion expression is the same as that depicted in FIG. 10, descriptions thereof are omitted. An example of detection of a predetermined motion is performed by the same method as that described in the first example and descriptions thereof are omitted.

FIG. 29 is an explanatory diagram depicting an example of quantification of a sensor waveform in the second example. The feature-amount calculating unit 407 calculates a feature amount from the sensor waveform in the detected motion interval to quantify the predetermined motion. When the predetermined motion is standing, the feature amount of standing is, for example, a forward bending speed at the time of standing or a standing time.

Reference numeral 2901 depicted in FIG. 29 denotes a sensor waveform that is in the detected motion interval and that is a part of the graph 2801-3. Reference numeral 2902 denotes a part of the sensor waveform in the detected motion interval and indicates which part of the sensor waveform corresponds to the forward bending speed. As indicated by reference numeral 2902, the forward bending speed corresponds to the amplitude of the sensor waveform. Reference numeral 2903 denotes a part of the sensor waveform in the detected motion interval and indicates which part of the sensor waveform corresponds to the standing time. As indicated by reference numeral 2903, the standing time corresponds to a time interval of one wave in the sensor waveform. The feature-amount calculating unit 407 accumulates the calculated feature amount in the feature amount database 415.

A feature amount database (standing) 415S depicted in FIG. 29 stores the calculated feature amount of walking therein. The feature amount database (standing) 415S depicted in FIG. 29 has records 2904-1 and 2904-2.

The feature amount database (standing) 415S includes fields for the date and time, the forward bending speed, the standing time, ⋯. Values indicating the date and time when the sensors 102 performed measurement are stored in the date-and-time field. Values indicating the calculated forward bending speed are stored in the forward-bending-speed field. Values indicating the calculated standing time are stored in the standing-time field.

As described above, the information processing apparatus 101 converts the sensor waveform for an affected part using a conversion method that maximizes the similarity between the sensor waveform for the affected part and the sensor waveform for the cooperating part when a patient performs a predetermined motion. This enables the information processing apparatus 101 to apply general quantification of the predetermined motion. Therefore, a person in charge of treatment of a patient does not need to perform adjustment of the sensors 102 according to recovery of the affected part or correction of the sensor waveform for the affected part. Accordingly, quantification can be performed easily. Alternatively, the information processing apparatus 101 can perform conversion of the sensor waveform and transmit the converted sensor waveform to another device that performs general quantification of the predetermined motion.

The information processing apparatus 101 can calculate the feature amount of the predetermined motion of the affected part on the basis of the converted sensor waveform for the affected part. Accordingly, the information processing apparatus 101 can apply general quantification of the predetermined motion and a person responsible for the patient does not need to perform adjustment of the sensors 102 according to recovery of the affected part or correction of the sensor waveform for the affected part. Therefore, quantification can be performed easily.

The information processing apparatus 101 can calculate the motion conversion amount and accumulate the calculated motion conversion amount. By monitoring the motion conversion amount, a person in charge of treatment of a patient can know the degree of change in the movement on the affected part according to an elapse of time, that is, the degree of recovery of the affected part over time from the degree of fluctuations in the motion conversion amount.

The information processing apparatus 101 can calculate the motion insufficiency from the motion conversion amount and convert the sensor waveform for the affected part on the basis of the motion insufficiency. This enables the information processing apparatus 101 to apply general quantification of the predetermined motion with consideration of the motion insufficiency with respect to the predetermined motion such as deceleration, bend, or rotation.

The information processing apparatus 101 can determine the cooperating part on the basis of the sensor waveform for the affected part and sensor waveforms of a plurality of parts different from the affected part. Accordingly, the information processing apparatus 101 can suppress erroneous conversion using the sensor waveform of a movement unrelated to a movement of the affected part produced with the predetermined motion.

The information processing apparatus 101 can determine, as the cooperating part, a part having a motion duration in which an overlap with the motion duration of the affected part due to the predetermined motion is long among a plurality of parts. This enables the information processing apparatus 101 to determine, as the cooperating part, a part in which the movement continues with the affected part during execution of the predetermined motion. Therefore, erroneous conversion can be suppressed.

When the predetermined motion is walking of a person and the affected part is either one of two legs of a patient, the information processing apparatus 101 can determine the other one of the two legs of the patient as the cooperating part. When the predetermined motion is standing of a person and the affected part is the waist of the person, the cooperating part can be a leg of the person or a wrist of the person. When an appropriate cooperating part is thus known in advance, the restriction of the cooperating part can suppress labor to attach unnecessary sensors 102 or suppress load to obtain sensor waveforms.

The information processing apparatus 101 can perform conversion only for the rotation amount on a horizontal plane when the predetermined motion is walking of a person and the affected part is either one of two legs of a patient. The information processing apparatus 101 can perform conversion only for the forward/backward rotation amount of a leg or a wrist when the predetermined motion is standing of a person and the affected part is the waist of the person. When the rotation direction of conversion is thus previously known, the information processing apparatus 101 can limit the rotation direction of the conversion and suppress load of processing to calculate the motion conversion amount or the motion insufficiency.

The information processing apparatus 101 can determine the waist of a person as the cooperating part when the predetermined motion is walking of a person and the affected part is either one of two legs of the person. This enables the information processing apparatus 101 to detect the feature amount of walking even from the sensor waveform of the affected part of a patient having semi-permanent motion abnormality in one leg such as hemiparesis.

The information processing method explained in the present embodiment may be implemented by a computer, such as a personal computer and a workstation, executing a program that is prepared in advance. The information processing program is recorded on a computer-readable recording medium such as a hard disk, a flexible disk, a CD-ROM, an MO, and a DVD, and is executed by being read out from the recording medium by a computer. The program may be distributed through a network such as the Internet.

EXPLANATIONS OF LETTERS OR NUMERALS

101    processing apparatus
402    motion-expressing-sensor determining unit
403    motion-conversion-amount calculating and accumulating unit
404    motion-conversion-amount extracting unit
405    motion converting unit
406    motion detecting unit
407    feature-amount calculating unit
410    sensor-data storage unit
412    motion-expression-index definition database
413    motion-conversion-amount database
414    motion-conversion definition database
415    feature amount database

**Claims**

1.  An information processing apparatus comprising:

    a control unit configured to:

        determine a conversion method for converting first data obtained from a sensor that measures a movement of a certain part occurring with a predetermined motion, the conversion method being determined so as to maximize similarity between time-series first data and time-series second data obtained from another sensor that measures a movement of a cooperating part occurring with the predetermined motion, the cooperating part moving in cooperation with the certain part, and
convert the first data based on the determined conversion method.

2. The information processing apparatus according to claim 1, wherein the control unit is configured to calculate a feature amount indicating a feature of the predetermined motion for the certain part, based on the converted first data.

3. The information processing apparatus according to claim 1 or 2, wherein the control unit is configured to:

input the first data and the second data to a predetermined conversion expression including parameters to determine values of the parameters so as to maximize similarity between the first data and the second data, and accumulate the determined values of the parameters in a storage unit.

4. The information processing apparatus according to claim 3, wherein the control unit is configured to:

calculate a deviation amount of a movement of the certain part from the predetermined motion, based on the values accumulated in the storage unit, and
convert the first data, based on the calculated deviation amount.

5. The information processing apparatus according to any one of claims 1 to 4, wherein the control unit is configured to:

determine the cooperating part from parts different from the certain part, based on the first data and time-series data respectively obtained from sensors that measure movements of the parts, and
determine the conversion method so as to maximize similarity between the first data and the time-series second data obtained from the sensor that measures the movement of the determined cooperating part.

6. The information processing apparatus according to claim 5, wherein the control unit is configured to determine the cooperating part from the parts, based on a length of an overlap of a period in which the certain part moves due to the predetermined motion, the period being specified from the first data, with periods in which the parts respectively move due to the predetermined motion, the periods being specified from the time-series data respectively obtained from the sensors that measure the movements of the parts.

7. The information processing apparatus according to any one of claims 1 to 5, wherein, when the predetermined motion is walking of a person and the certain part is one of two legs of the person, another leg of the two legs is determined as the cooperating part.

8. The information processing apparatus according to claim 3, wherein
the predetermined motion is walking of a person, the certain part is one of two legs of the person, and the cooperating part is another leg of the two legs, and
the control unit is configured to input the first data and the second data to a predetermined conversion expression including as a parameter, a rotation amount on a horizontal plane to determine a value of the rotation amount so as to maximize similarity between the first data and the second data, as the conversion method.

9. The information processing apparatus according to any one of claims 1 to 5, wherein, when the predetermined motion is standing of a person and the certain part is a waist of the person, a leg of the person or a wrist of the person is determined as the cooperating part.

10. The information processing apparatus according to claim 3, wherein
the predetermined motion is standing of a person, the certain part is a waist of the person, and the cooperating part is a leg of the person or a wrist of the person, and
the control unit is configured to input the first data and the second data to a predetermined conversion expression including as a parameter, a forward/backward rotation amount of the leg or the wrist to determine a value of the rotation amount so as to maximize similarity between the first data and the second data, as the conversion method.

11. The information processing apparatus according to any one of claims 1 to 5, wherein, when the predetermined motion is walking of a person and the certain part is one of two legs of the person, a waist of the person is determined as the cooperating part.

12. An information processing method executed by a computer, the information processing method comprising:

determining a conversion method for converting first data obtained from a sensor that measures a movement of a certain part occurring with a predetermined motion, the conversion method being determined so as to maximize similarity between time-series first data and time-series second data obtained from another sensor that measures a movement of a cooperating part occurring with the predetermined motion, the cooperating part moving in cooperation with the certain part; and
converting the first data based on the determined conversion method.

13. An information processing program that causes a computer to execute a process comprising:

determining a conversion method for converting time-series first data obtained from a sensor that measures a movement of a certain part occurring with a predetermined motion, the conversion method being determined so as to maximize similarity between the first data and time-series second data obtained from a sensor that measures a movement of a cooperating part occurring with the predetermined motion, the cooperating part moving in cooperation with the certain part; and
converting the first data based on the determined conversion method.

FIG.1

103t1-$X_a$

103t2-$X_a$

103t3-$X_a$

uA

$102_n$

$102_a$

$102_n$

$102_a$

$102_n$

$102_a$

101

INFORMATION PROCESSING APPARATUS

103t1-$X_a$

(2)
CONVERSION

103t1-$X'_a$

103t1-$X_n$

(1) DETERMINE CONVERSION METHOD

FIG.2

FIG.3

# FIG.4

**DATA OBTAINING APPARATUS** ⌐201

SENSOR-DATA OBTAINING UNIT ⌐401

SENSOR-DATA STORAGE UNIT ⌐410

200

⌐101

**INFORMATION PROCESSING APPARATUS**

STORAGE UNIT ⌐411

MOTION-EXPRESSION-INDEX DEFINITION DATABASE ⌐412

MOTION-CONVERSION-AMOUNT DATABASE ⌐413

MOTION-CONVERSION DEFINITION DATABASE ⌐414

FEATURE AMOUNT DATABASE ⌐415

CONTROL UNIT ⌐301

MOTION-EXPRESSING-SENSOR DETERMINING UNIT 402

MOTION-CONVERSION-AMOUNT CALCULATING AND ACCUMULATING UNIT 403

MOTION-CONVERSION-AMOUNT EXTRACTING UNIT ⌐404

MOTION CONVERTING UNIT 405

MOTION-DETECTING UNIT ⌐406

FEATURE-AMOUNT CALCULATING UNIT ⌐407

FIG.5

## FIG.6

# FIG.7

SENSOR 102-1 AT AFFECTED PART (LEFT LEG)

MOTION START TIME OF $T_1$      MOTION END TIME OF $T_1$

$t_{1,s}$      $t_{1,e}$    $T_1$   701

SENSOR 102-2 (RIGHT LEG)

$t_{2,s}$      $t_{2,e}$    $T_2$   702

SENSOR 102-3 (RIGHT HAND)

703

SENSOR 102-4 (LEFT HAND)

$t_{4,s}$    $t_{4,e}$    $T_4$   704

# FIG.8

AVERAGE VALUE OF PEAK VALUES
OF COMPOSITE ACCELERATION OF
AFFECTED PART: $Acc_a$

DIFFERENCE IN AMPLITUDE BETWEEN
SENSOR WAVEFORM FOR AFFECTED
PART AND SENSOR WAVEFORM OF
MOTION EXPRESSING SENSOR:
$W = Acc_n - Acc_a$

AVERAGE VALUE PEAK VALUES OF
COMPOSITE ACCELERATION OF
MOTION EXPRESSING SENSOR: $Acc_n$

# FIG.9

SENSOR WAVEFORM FOR
AFFECTED PART: $X_a$
901

$$
\begin{array}{l}
400 \\
200 \\
0 \\
-200 \\
-400
\end{array}
$$

WAVEFORM OBTAINED BY ROTATION
CONVERSION OF SENSOR WAVEFORM
FOR AFFECTED PART:
$X_a\cos(\frac{\pi}{2})-Z_a\sin(\frac{\pi}{2})$

$$
\begin{array}{l}
400 \\
200 \\
0 \\
-200 \\
-400
\end{array}
$$
902

SENSOR WAVEFORM OF
MOTION EXPRESSING SENSOR: $X_n$
903

$$
\begin{array}{l}
400 \\
200 \\
0 \\
-200 \\
-400
\end{array}
$$

# FIG.10

SENSOR WAVEFORM
FOR AFFECTED PART: $X_a$

400
200
0
-200
-400

1001

CONVERSION WITH
RESPECT TO ROTATION
$X'_a = X_a\cos\Phi - Z_a\sin\Phi$

400
200
0
-200
-400

1002

CONVERSION WITH
RESPECT TO
DECELERATION AND BEND
$\hat{X}_a = W \cdot X'_a$

400
200
0
-200
-400

1003

# FIG.11

## FIG.12

SENSOR WAVEFORM FOR AFFECTED PART ⌐1201

SENSOR WAVEFORM AFTER CONVERSION ⌐1203

SENSOR WAVEFORM OF
MOTION EXPRESSING SENSOR ⌐1202

FIG.13

# FIG.14

START

OBTAIN SENSOR DATA — S1401

DETERMINE MOTION EXPRESSING SENSOR — S1402

CALCULATE/ACCUMULATE MOTION CONVERSION AMOUNT FOR AFFECTED PART SO AS TO MAXIMIZE SIMILARITY BETWEEN SENSOR WAVEFORM FOR AFFECTED PART AND SENSOR WAVEFORM OF MOTION EXPRESSING SENSOR — S1403

CALCULATE MOTION INSUFFICIENCY FROM MOTION CONVERSION AMOUNT — S1404

CONVERT SENSOR WAVEFORM FOR AFFECTED PART ON BASIS OF CALCULATED MOTION INSUFFICIENCY — S1405

DETECT/QUANTIFY PREDETERMINED MOTION OF AFFECTED PART — S1406

END

FIG.15

MANNER OF WALKING OF PATIENT HAVING SPRAIN

uA    uA    uA

201    201    201

201    201    201    201

201    201

...

201    201    201

201    201    201

→ TIME

SENSOR
-DATA
OBTAIN-
ING
UNIT    401

SENSOR-DATA
STORAGE
UNIT    410

FIG.16

LEFT LEG
(AFFECTED
PART)

RIGHT LEG

WAIST
(POSTERIOR)

WAIST
(ANTERIOR)

LEFT WRIST

RIGHT WRIST

1601-1

1601-2

1601-3

1601-4

1601-5

1601-6

TIME

401

SENSOR-
DATA
OBTAINING
UNIT

410

SENSOR-DATA
STORAGE UNIT

# FIG.17

| DEFINI-TION | AFFECTED PART | MOTION EXPRESSION INDEX | | | |
|---|---|---|---|---|---|
| | | COOPERAT-ING PART | COOPERATING-PART DETER-MINING INDEX | FOCUSED FEATURE AMOUNT | |
| (1) | LEFT LEG | RIGHT LEG | - | - | ~1701-1 |
| (2) | LEFT LEG | - | MOTION DURATION | X-AXIS GYROSCOPE VALUE | ~1701-2 |
| (3) | LEFT LEG | - | MOTION DURATION | COMPOSITE SENSOR VALUE | ~1701-3 |
| ... | ... | ... | ... | ... | |
| (10) | WAIST (POSTERIOR) | RIGHT WRIST | - | - | ~1701-10 |
| (11) | WAIST (POSTERIOR) | - | MOTION DURATION | X-AXIS GYROSCOPE VALUE | ~1701-11 |
| ... | ... | ... | ... | ... | |

412

MOTION-EXPRESSION-INDEX DEFINITION DATABASE

402

MOTION-EXPRESSING-SENSOR DETERMINING UNIT

FIG.18

# FIG.19

MOTION RATIO: $T_{mot}$

COMPOSITE VALUE OF SENSOR DATA OF AFFECTED PART: $R_a$

COMPOSITE VALUE OF SENSOR DATA OF COOPERATING PART: $R_n$

ROTATION AMOUNT: $T_{rot}$

SENSOR DATA OF AFFECTED PART: $D_a$

SENSOR DATA OF COOPERATING PART: $D_n$

413

MOTION-CONVERSION-AMOUNT DATABASE

403

MOTION-CONVERSION-AMOUNT CALCULATING AND ACCUMULATING UNIT

MOTION CONVERSION AMOUNT THAT MAXIMIZES SIMILARITY: $\hat{T} = \arg\min_{T} \| U_n - T U_a \|$

$$\hat{T} = \arg\min_{T} \left\| \begin{pmatrix} R_n \\ D_n \\ \vdots \end{pmatrix} - \begin{pmatrix} T_{mot} & 0 & \cdots \\ 0 & T_{rot} & \cdots \\ \vdots & \vdots & \ddots \end{pmatrix} \begin{pmatrix} R_n \\ D_n \\ \vdots \end{pmatrix} \right\|$$

# FIG.20

| DATE AND TIME | AFFECTED PART | COOPE-RATING PART | MOTION CONVERSION AMOUNT | | |
| --- | --- | --- | --- | --- | --- |
| | | | MOTION RATIO | ROTATION AMOUNT | ... |
| 2015/11/24 | LEFT LEG | RIGHT LEG | 1.40 | 90 | ... |
| ... | ... | ... | ... | ... | ... |
| 2015/11/24 | WAIST (POSTERIOR) | RIGHT WRIST | 2.34 | 52 | ... |
| ... | ... | ... | ... | ... | ... |

~2001-1

~2001-2

413

MOTION-CONVERSION-AMOUNT DATABASE

403

MOTION-CONVERSION-AMOUNT CALCULATING AND ACCUMULAT-ING UNIT

## FIG.21

**MOTION-CONVERSION DEFINITION DATABASE** ~414

| DEFINITION | AFFECTED PART | COOPERAT-ING PART | REQUIRED CONVERSION AMOUNT | | | |
|---|---|---|---|---|---|---|
| | | | MOTION RATIO | ROTATION AMOUNT | - | ~2101-a |
| a | LEFT LEG | RIGHT LEG | MOTION RATIO | ROTATION AMOUNT | - | |
| ... | ... | ... | ... | ... | ... | |
| f | WAIST (POSTERIOR) | RIGHT WRIST | MOTION RATIO | ROTATION AMOUNT | - | ~2101-f |
| ... | ... | ... | ... | ... | ... | |

**MOTION-CONVERSION-AMOUNT DATABASE** ~413

| DATE AND TIME | AFFECTED PART | COOPERAT-ING PART | MOTION CONVERSION AMOUNT | | | |
|---|---|---|---|---|---|---|
| | | | MOTION RATIO | ROTATION AMOUNT | ... | |
| 2015/11/24 | LEFT LEG | RIGHT LEG | 1.40 | 90 | ... | ~2001-1 |
| ... | ... | ... | ... | ... | ... | |
| 2015/11/24 | WAIST (POSTERIOR) | RIGHT WRIST | 2.34 | 52 | ... | ~2001-3 |
| ... | ... | ... | ... | ... | ... | |

413
MOTION-CONVERSION-AMOUNT DATABASE

414
MOTION-CONVERSION DEFINITION DATABASE

404
MOTION-CONVERSION-AMOUNT EXTRACTING UNIT

# FIG.22

SENSOR WAVEFORM FOR AFFECTED PART: $X_a$

BEFORE CONVERSION

2201

CONVERSION FOR ROTATION AMOUNT:
$X'_a = X_a \cos\Phi - Z_a \sin\Phi$

2202

CONVERSION FOR MOTION RATIO:
$\hat{X}_a = W \cdot X'_a$

AFTER CONVERSION

2203

405

MOTION CONVERTING UNIT

FIG.23

FIG.24

## FIG.25

MANNER IN WHICH HEMIPARETIC PATIENT STANDS UP

TIME

SENSOR-DATA OBTAINING UNIT
401

SENSOR-DATA STORAGE UNIT
410

FIG.26

LEFT LEG — 2601-1

50

RIGHT LEG — 2601-2

50

WAIST (POSTERIOR) (AFFECTED PART) — 2601-3

50

WAIST (ANTERIOR) — 2601-4

50

LEFT WRIST — 2601-5

50

RIGHT WRIST — 2601-6

SENSOR-DATA OBTAINING UNIT — 401

SENSOR-DATA STORAGE UNIT — 410

## FIG.27

FIG.28

BEFORE
CONVERSION

SENSOR WAVEFORM FOR AFFECTED PART: $X_a$

~ 2801

CONVERSION FOR ROTATION AMOUNT:
$X'_a = X_a \cos\Phi - Z_a \sin\Phi$

~ 2802

AFTER
CONVERSION

CONVERSION FOR MOTION RATIO:
$\hat{X}_a = W \cdot X'_a$

~ 2803

405

MOTION
CONVERTING
UNIT

FIG.29

## INTERNATIONAL SEARCH REPORT

| International application No. |
|---|
| PCT/JP2016/061669 |

**A. CLASSIFICATION OF SUBJECT MATTER**
*A61B5/11(2006.01)i*

According to International Patent Classification (IPC) or to both national classification and IPC

**B. FIELDS SEARCHED**

Minimum documentation searched (classification system followed by classification symbols)
A61B5/06-5/22

Documentation searched other than minimum documentation to the extent that such documents are included in the fields searched
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)
JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 2011-177300 A (Empire Technology Development LLC), 15 September 2011 (15.09.2011), entire text; all drawings & US 2011/0213582 A1 | 1–13 |

☐ Further documents are listed in the continuation of Box C.   ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 17 June 2016 (17.06.16) | 28 June 2016 (28.06.16) |

| Name and mailing address of the ISA/ Japan Patent Office 3-4-3,Kasumigaseki,Chiyoda-ku, Tokyo 100-8915,Japan | Authorized officer |
|---|---|
|  | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011177300 A
- JP 2011092696 A
- JP 2012516719 W

- JP 2002065640 A
- JP 2012055604 A
- JP 2012090651 A

**Non-patent literature cited in the description**

- **PAOLO FRACCARO.** Real-world Gyroscope-based Gait Event Detection and Gait Feature Extraction. *eTELEMED,* 2014

- **VAN LUMMEL.** Automated approach for quantifying the repeated sit-to-stand using one bodyfixed sensor in young and older adults. *Gait & Posture,* 2013, vol. 38, 153-156